# EUROPEAN PATENT SPECIFICATION

(11) **EP 3 680 663 B1**
(45) Date of publication and mention of the grant of the patent: **06.12.2023**
(21) Application number: 18853298.0
(22) Date of filing: 07.09.2018
(51) Int. Cl.: G01N 33/543, G01N 33/545, G01N 37/00

(54) **SUBSTRATE FOR BIOCHIP, BIOCHIP, METHOD FOR MANUFACTURING BIOCHIP, AND METHOD FOR PRESERVING BIOCHIP**
SUBSTRAT FÜR BIOCHIP, BIOCHIP, VERFAHREN ZUR HERSTELLUNG EINES BIOCHIPS UND VERFAHREN ZUR KONSERVIERUNG EINES BIOCHIPS
SUBSTRAT POUR BIOPUCE, BIOPUCE, PROCÉDÉ DE FABRICATION DE BIOPUCE ET PROCÉDÉ DE CONSERVATION DE BIOPUCE

(30) Priority: 07.09.2017 JP 2017172453; 07.09.2017 JP 2017172454
(43) Date of publication of application: 15.07.2020
(73) Proprietor: Mitsubishi Gas Chemical Company, Inc., Tokyo 100-8324 (JP)
(72) Inventor: SAWANO Erika, Tokyo 100-8324 (JP); TAKASE Yuki, Tokyo 100-8324 (JP); TASHIRO Hideo, Tokyo 113-0024 (JP); TASHIRO Tomoko, Tokyo 113-0024 (JP)
(74) Representative: Müller-Boré & Partner Patentanwälte PartG mbB
(86) International application number: PCT/JP2018/033301
(87) International publication number: WO 2019/050017

(56) References cited:
- WO-A1-03/000433
- WO-A1-2005/093416
- WO-A1-2007/043748
- GB-A- 2 422 335
- JP-A- S6 396 558
- JP-A- S63 151 857
- JP-A- 2005 121 443
- JP-A- 2006 282 871
- JP-A- 2007 139 587
- JP-A- 2008 002 974
- JP-A- 2008 275 371
- JP-A- 2010 101 661
- JP-A- 2013 011 479
- JP-A- 2013 011 479
- JP-A- 2016 079 397
- US-A1- 2016 362 781

## Description

The present invention relates to a biochip used in the detection or analysis of a biochemical reaction, etc., utilizing a biological material immobilized on a substrate, a method for producing the biochip, and a package prepared by packaging the biochip. The present invention particularly relates to a biochip for inspection and diagnosis, which utilizes an antigen-antibody reaction and is used in allergy tests, tumor marker tests, and the like.

In recent years, an immunoplate for immunoassay, in which an antibody or an antigen is immobilized on a plate, a DNA chip in which a nucleic acid is immobilized on a chip, and the like have been broadly used. In these chips, biological materials such as proteins or nucleic acids that are used as materials to be immobilized are immobilized, in the form of spots, on the surface of a substrate as a microarray.

As methods of immobilizing a protein or the like on a substrate, a method comprising spotting BSA on a polymer layer obtained by applying a vinyl polymer of polyethylene glycol monomethacrylate having a polyethylene glycol moiety with a molecular weight of 350 onto an acrylic resin substrate, and then immobilizing the BSA on the substrate according to a photocrosslinking reaction using sodium 4,4'-diazidostilbene-2,2'-disulfonate (Patent Literature 1), and a method comprising spotting N-[4-[3-(trifluoromethyl)-3H-diazirin-3-yl]phenyl]oxirane and peptides on a film obtained by applying a vinyl polymer of polyethylene glycol monomethacrylate having a polyethylene glycol moiety with a molecular weight of 350 onto an acrylic resin substrate, and then immobilizing the peptides thereon (Patent Literature 2) have been disclosed.

On the other hand, as a method of performing the detection or analysis of a biochemical reaction, a method comprising introducing maleimide groups in the form of spots into PEG₃-OH alkanethiol bound to a chromium- and gold-evaporated glass slide by using 8-AOT and SSMCC, then aligning and immobilizing peptides in the form of spots via the maleimide groups, and then using the thus formed array to supply a test sample to an SPR device, so as to detect phosphorylation of cSrc kinase, has been disclosed (Patent Literature 3).

Patent Literature 4 discloses a method comprising performing an activation treatment on a silicon- or stainless steel-made microchip coated with diamond/diamond-like carbon, then spotting allergen thereon, inactivating unreacted groups remaining on the chip, further blocking with BSA, and then allowing the chip to react with a test sample.

As a chip capable of detecting a small amount of test sample, a chip comprising, on a base material, a plurality of reaction chambers (wells), flow channels connecting with each of the above-described wells, and injection ports for injecting a solution into the above-described flow channels, wherein the chip is capable of analyzing a plurality of test samples or performng a reaction with a small amount of test sample, is disclosed (Patent Literature 5). Moreover, a chip comprising a spot on which a component reacting with a detection target component is immobilized, wherein the spot is disposed in a storage portion capable of storing a liquid test sample, a drainage port for discharging a test sample and a washing solution by centrifugal force, and a separation port, is disclosed (Patent Literature 6).

Moreover, since a protein chip, in which a protein is immobilized on a plate, or the like has been generally developed as an extension of a DNA chip, studies have been conducted regarding immobilization of a protein on a glass substrate, or immobilization of a molecule capturing the protein or the like (i.e., a material to be immobilized) on the surface of a chip (Patent Literature 7).

After a material to be immobilized has been immobilized on a substrate, the material to be immobilized is allowed to react with another physiologically active substance (a material to be detected; a protein, an antigen, etc.) on the surface of the substrate, and a labeled protein is allowed to further react with the reaction mixture, and finally, detection is carried out using a detector or the like. In such a case, if a material to be detected other than a capture molecule is immobilized on a portion, on which the capture molecule has not been immobilized, it makes noise upon detection, and causes a decrease in the signal to noise ratio (S/N ratio), thereby reducing detection accuracy. In particular, non-specific adsorption of contaminant proteins is often found in serum or plasma used in clinical diagnosis and the like, and thus, high noise is made, and the S/N ratio thereby tends to be decreased.

Thus, in order to prevent the non-specific adsorption of an antigen and a secondary antibody after immobilization of an antibody on the surface of a chip, the chip is coated with a non-specific adsorption preventing agent. For example, a method of applying a vinyl polymer of polyethylene glycol monomethacrylate having a polyethylene glycol moiety with a molecular weight of 350 onto an acrylic resin substrate (Patent Literature 2) and a method of binding PEG₃-OH alkanethiol onto a chromium- and gold-evaporated glass slide (Patent Literature 3) are disclosed.

On the other hand, there is disclosed a substrate for immobilization of a physiologically active substance, in which a layer comprising a polymer constituted with 2-methacryloyloxyethyl phosphorylcholine, n-cyclohexyl methacrylate, and N-[2-[2-[2-(t-butoxycarbonylaminooxyacetylamino)ethoxy]ethoxy]ethyl]-methac rylamide is introduced onto a saturated cyclic polyolefin resin substrate, so that aldehyde groups or maleimide groups are provided on the surface to immobilize a peptide thereon, and the aldehyde groups or maleimide groups remaining on the surface are then inactivated, so that the non-specific adsorption/binding of a material to be detected can be suppressed without coating the surface with an absorption-preventing agent (Patent Literature 8).

Patent Literature 1: JP Patent Publication (Kokai) No. 2006-322708 A
Patent Literature 2: JP Patent Publication (Kokai) No. 2010-101661 A
Patent Literature 3: JP Patent Publication (Kokai) No. 2007-24729 A
Patent Literature 4: Japanese Patent No. 5322240
Patent Literature 5: Japanese Patent No. 4962658
Patent Literature 6: JP Patent Publication (Kokai) No. 2016-6414 A
Patent Literature 7: JP Patent Publication (Kokai) No. 2001-116750 A
Patent Literature 8: JP Patent Publication (Kokai) No. 2010-117189 A

Further, JP 2013 011479 A describes a carrier for immobilizing a material for use in an immunoassay, WO 03/000433 A1 describes a functional surface coating, GB 2 422 335 A describes a biochip, JP 2016 079397 A describes a hydrophilic treatment agent, US 2016/362781 A1 describes a method of modifying a substrate surface, and JP 2006 282871 A describes a method for keeping the hydrophilic property of a surface of a hydrophobic polymer substrate.

However, since the methods disclosed in Patent Literatures 1 and 2 cannot control the immobilized site of a material to be immobilized, these methods have been problematic in that the detection efficiency of a material to be detected is low, and further, in that uniform spots can be hardly obtained and detection sensitivity is not high. In addition, the method disclosed in Patent Literature 3 has been problematic in that it is difficult to conduct a test with a small amount of test sample. On the other hand, in the case of the chip disclosed in Patent Literature 4, it may be possible to conduct a test with a small amount of test sample, but it requires a special substrate coated with diamond/diamond-like carbon. What is more, after an activation treatment has been performed on the chip of Patent Literature 4, allergen is spotted thereon, and then, unreacted groups remaining on the chip are inactivated, and further, it must be blocked with BSA. Thus, preparation of this chip is complicated. The chips disclosed in Patent Literatures 5 and 6 have been problematic in that the step of molding the chips becomes complicated because the chips have complicated shapes, and have also been problematic in that manufacturing failures often take place.

Moreover, the methods of Patent Literatures 2 and 3 require a step of forming, on a substrate, a coating layer for preventing the non-specific adsorption of a material to be detected on a substrate and immobilizing a biological material on the substrate. However, such a step of forming the above-described coating layer on a substrate requires time and effort or costs, and defective products are easily generated. Accordingly, these methods are not necessarily satisfactory. On the other hand, on the substrate of Patent Literature 8, a coating layer for preventing non-specific adsorption is not formed, but a layer for immobilizing a material to be immobilized is formed, and after such a material to be immobilized has been immobilized, a hydrophobization treatment is performed to prevent non-specific adsorption. Accordingly, this step is more complicated than the steps disclosed in Patent Literatures 2 and 3.

Furthermore, since these chips have been used for immobilization of biological materials, refrigeration or freezing has been necessary for preservation of the chips.

As such, it has been desired to develop a biochip, in which the aforementioned problems of the prior art techniques have been improved.

The above object underlying the present invention has been solved by providing the subject matter as set out in the appended claims.

In particular, the present invention provides a biochip having a substrate with a hydrophilic reaction area and a spot of a material to be immobilized comprising a biological material that are disposed in the reaction area,
wherein the spot further comprises a thickener and a surfactant,
wherein the contact angle of the hydrophilic reaction area measured by a static contact angle measurement method is 1° or more and less than 80°,
wherein the thickener is gellan gum, xanthan gum, curdlan, pullulan, a guar gum derivative, locust bean gum, carrageenan, pectin, tamarind gum, psyllium seed gum, dextran, glycerin, carboxymethyl cellulose, hydroxyethyl cellulose, hydroxymethylpropyl cellulose, lanolin, methyl cellulose, Vaseline, polyethylene glycol, polyvinyl alcohol, polyvinyl pyrrolidone, a carboxyl vinyl polymer, polyvinyl pyrrolidone, polyvinyl alcohol, dextrin acid fatty ester, inulin acid fatty ester, or a mixture of two or more types selected from these,
wherein the surfactant is a nonionic surfactant, and
wherein the spot is formed by
a step of allowing a liquid comprising the material to be immobilized, a photocrosslinking agent having at least two photoreactive groups in a single molecule, and the thickener and the surfactant, to adhere, in the form of grid-like spots, to the substrate; and
a step of immobilizing the material to be immobilized adhering to the substrate thereon.

The biochip of the present invention has one or two or more of the following effects.
(1) The biochip enables analysis with a small amount of test sample.
(2) The binding site of a material to be immobilized can be controlled.
(3) The biochip has high uniformity in terms of the distribution of materials to be immobilized in a spot.
(4) The biochip has an excellent S/N ratio.
(5) The biochip has excellent detection sensitivity.
(6) The biochip enables high-speed analysis.
(7) The biochip enables the immobilization of a material to be immobilized without being coated with a coating layer such as a polymer.
(8) The biochip has excellent luminescence intensity.
(9) The biochip can suppress the non-specific adsorption of a material to be detected.
(10) The biochip has high sensitivity.
(11) The biochip can be preserved at normal temperature.
(12) The production process of the biochip is simple.
(13) The biochip provides inexpensive analysis costs.
(14) The biochip is easily handled.
(15) It is possible to apply the biochip to point of care testing analysis.

### Brief Description of Drawings

Figure 1 is an explanatory view showing one aspect of the biochip of the present invention. Figure 1(a) is a plan view, and Figure 1(b) is a cross-sectional view formed by cutting Figure 1(a) with the line A-A'.
Figure 2 is an explanatory view showing another aspect of the biochip of the present invention. Figure 2(a) is a plan view, and Figure 2(b) is a cross-sectional view formed by cutting Figure 2(a) with the line A-A'.
Figure 3 is an explanatory view showing another aspect of the biochip of the present invention. Figure 3(a) is a plan view, and Figure 3(b) is a cross-sectional view formed by cutting Figure 3(a) with the line A-A'.
Figure 4 is an explanatory view showing another aspect of the biochip of the present invention. Figure 4(a) is a plan view, and Figure 4(b) is a cross-sectional view formed by cutting Figure 4(a) with the line A-A'.
Figure 5 is a photographic view including the photomicrographs of individual spots of the biochip of Example 3. The number at the upper left of each photograph indicates the number of a stamp solution.
Figure 6 is a view showing the measurement results of the luminescence intensity obtained by detection using the biochip of Example 4. The left is a photographic view showing the luminescence intensity of each spot of the chip, and the right indicates each spot number. To the spots having the same spot number, the same stamp solution is applied.
Figure 7 is a view showing the results obtained by observing the spot indicated with " 1" in Figure 6, using a laser microscope. The left is a planar image, the center is a 3D image, and the right is a profile graph.
Figure 8 is a view showing the results obtained by observing the spot indicated with "2" in Figure 6, using a laser microscope. The left is a planar image, the center is a 3D image, and the right is a profile graph.
Figure 9 is a view showing the results obtained by observing the spot indicated with "3" in Figure 6, using a laser microscope. The left is a planar image, the center is a 3D image, and the right is a profile graph.
Figure 10 is a view showing the results obtained by observing the spot indicated with "4" in Figure 6, using a laser microscope. The left is a planar image, the center is a 3D image, and the right is a profile graph.
Figure 11 is a view showing the results obtained by observing the spot indicated with "5" in Figure 6, using a laser microscope. The left is a planar image, the center is a 3D image, and the right is a profile graph.
Figure 12 is a view showing the results obtained by observing the spot indicated with "6" in Figure 6, using a laser microscope. The left is a planar image, the center is a 3D image, and the right is a profile graph.
Figure 13 is a view showing the results obtained by observing the spot indicated with "7" in Figure 6, using a laser microscope. The left is a planar image, the center is a 3D image, and the right is a profile graph.
Figure 14 is a view showing the results obtained by observing the spot indicated with "8" in Figure 6, using a laser microscope. The left is a planar image, the center is a 3D image, and the right is a profile graph.
Figure 15 is a view showing the measurement results of the luminescence intensity obtained by detection using the biochip of Example 5. The left is a photographic view showing the luminescence intensity of each spot of the chip, and the right indicates each spot number. To the spots having the same spot number, the same stamp solution is applied.
Figure 16 is a view showing the measurement results of the luminescence intensity obtained by detection using the biochips of Preservation Examples 1 to 3. The chips on the left side and upper right each show a photographic view of the luminescence intensity of each spot. The lower right indicates each spot number. To the spots having the same spot number, the same stamp solution is applied.
Figure 17 is a view showing the measurement results of the luminescence intensity obtained by detection using the biochips produced using Biochip Substrates 1 to 7.
Figure 18 is a view showing the measurement results of the luminescence intensity obtained by detection using the biochips of Preservation Examples 4 and 5.

Hereinafter, the present invention will be described with reference to the figures. It is to be noted that the same reference signs are assigned to constituent elements exhibiting the same or similar function in each figures, so that overlapped explanation is omitted.

### Biochip of the present invention

The biochip of the present invention is composed of, at least, a biochip substrate and a material to be immobilized, and has a reaction area for the biochemical reaction of a test sample on the upper surface of the substrate. In particular, the biochip of the present invention has a substrate with a hydrophilic reaction area and a spot of a material to be immobilized comprising a biological material that is disposed in the reaction area. The reaction area may be surrounded by a boundary capable of retaining a liquid therein.

The test sample, to which the biochip of the present invention can be applied, is not particularly limited. Examples of the test sample may include body fluids such as blood, plasma, serum, saliva, urine, lymph, cerebrospinal fluid, joint fluid, nasal discharge, ascites, aqueous humor and lacrimal fluid, sputum, and biopsy specimens, which become targets of allergy or tumor diagnosis or the like.

The substrate used in the biochip of the present invention is not particularly limited, as long as it does not excessively affect test samples or biochemical reactions. For example, a resin material or the like can be used. The type of the resin material is not limited, and for example, a thermosetting resin or a thermoplastic resin can be used. In particular, if a light transmissive resin material such as polypropylene, polycarbonate, acryl, polystyrene, polyethylene terephthalate, a cycloolefin polymer or a cycloolefin copolymer is used, favorable visible light transmittance can be ensured. The type of polypropylene is not limited, and for example, a random copolymer of homopolypropylene or polypropylene and polyethylene can be used. In addition, the acryl is not limited, and for example, a copolymer of methyl polymethacrylate or methyl methacrylate and a monomer such as methacrylic acid ester, acrylic acid ester or styrene can be used. Besides, the terms "transparence" and "light transmittance" are used in the present invention to mean that the average transmittance in the wavelength region of a detected light is 70% or more. If a light transmissive material is used in a visible light region (wavelength: 350 to 780 nm), the condition of a sample in the chip can be easily confirmed by visual observation, but is not limited thereto. The thickness of the substrate is not particularly limited. Since the substrate desirably has a certain extent of non-deforming property in the production process thereof, the thickness of the substrate is preferably 0.3 mm to 3.0 mm, more preferably 0.5 mm to 1.5 mm, and particularly preferably 0.7 mm to 1.0 mm.

In some aspect, a hydrophobic resin material is used in the substrate of the biochip of the present invention. In some aspect, the resin material is formed from a water-insoluble polymer. In some aspect, the resin material does not comprise dextran, polyethylene glycol, or a derivative thereof.

A hydrophilization treatment can be performed on the substrate. By performing such a hydrophilization treatment, non-specific adsorption on the substrate can be prevented, and the material to be immobilized can be strongly immobilized on the substrate. The term "immobilization" is used in the present description to mean that a strong chemical bond such as a covalent bond is formed between a material to be immobilized and the surface of a substrate, and thus, the immobilization in the present description is distinguished from a weak chemical bond (also referred to as chemical adsorption) such as a hydrogen bond, or physical adsorption due to Van der Waals force or the like. In addition, the term "non-specific adsorption" is used in the present description to mean that a material to be immobilized and/or a substance specifically reacting with the material to be immobilized are adsorbed on a region on the surface of a substrate, on which the material to be immobilized is not immobilized. When the material to be immobilized is, for example, an antigen, a specific example of the substance specifically reacting with the material to be immobilized may be an antibody. When the material to be immobilized is an antibody, a specific example of the substance specifically reacting with the material to be immobilized may be an antigen.

The hydrophilization treatment may be performed on one or several portions on the surface of the substrate, or may also be performed on the entire surface of the substrate. When the hydrophilization treatment is performed on a portion of the substrate surface, the treatment may be performed, for example, on the entire upper surface of the substrate, or on one or several portions on the upper surface of the substrate. The area, on which the hydrophilization treatment is performed, may be 10% or more, 20% or more, 30% or more, 40% or more, 50% or more, 60% or more, 70% or more, 80% or more, 90% or more, or the like, of the total surface area of the substrate or the total surface area of the upper surface of the substrate. Moreover, the portion, on which the hydrophilization treatment is performed, may have various shapes. For example, the portion, on which the hydrophilization treatment is performed, may have a shape such as a round shape, an oval shape, or a polygonal shape, and thereby, for example, it becomes possible to form a plurality of round-shape hydrophilic portions in the form of spots on the surface of the substrate.

The hydrophilization treatment is not particularly limited, but examples of the hydrophilization treatment may include: the coating of the surface with inorganic materials having hydrophilicity, such as silica, or surfactants; chemical hydrophilization treatments such as a plasma treatment, a UV-ozone treatment, a corona treatment, or a flame treatment; and the imparting of hydrophilicity by physically forming a fine nanostructure (WO2011/024947). When the substrate is made of a resin, it is more preferable to apply chemical hydrophilization treatments such as a plasma treatment, a UV-ozone treatment, a corona treatment, or a flame treatment, by which the chemical bonds of molecules on the surface of the resin can be cleaved, and functional groups having polarity, such as OH (hydroxyl groups), CO (carbonyl groups), COOH (carboxyl groups), methoxy groups, peroxide groups, or polar ether groups can be thereby generated depending on the type of the resin; and it is particularly preferable to apply a UV-ozone treatment. In addition, in one aspect, the hydrophilization treatment performed in the present invention does not involve the formation of a coating layer with a polymer, in particular, with a water-soluble polymer. Accordingly, the substrate of the biochip according to this aspect has a hydrophilic surface, but does not have a coating layer. Besides, the term "hydrophilicity" is used in the present invention to mean that the contact angle (θ/2 method) measured by a static contact angle measurement method (droplet method) is 1° or more and less than 80°, preferably 5° or more and less than 75°, and more preferably 10° or more and less than 70°.

In one aspect, the substrate of the biochip of the present invention has a surface comprising a hydrophilic reaction area, and preferably, a resin-made surface comprising a hydrophilic reaction area. The reaction area means a region on the substrate, on which a material to be immobilized is immobilized and is allowed to react with a detection target substance. The reaction area may be either a portion of the surface of the substrate, or the entire surface of the substrate. When the reaction area is a portion of the surface of the substrate, the surface of the substrate other than the reaction area may be either a hydrophilic or hydrophobic surface. When the reaction area is a portion of the surface of the substrate, the reaction area may have various shapes such as, for example, a round, oval, or polygonal shape, or a combination thereof. The reaction area may be either continuous or discontinuous. For example, the reaction area may form a plurality of spots that are not contacted with one another. The reaction area may be surrounded by a boundary capable of retaining a liquid therein. Otherwise, the reaction area may be covered with functional groups having polarity generated as a result that bonds associated with carbon of the resin are cleaved and the cleaved sites bind with oxygen. Examples of such a functional group include a hydroxyl group, a carbonyl group, a carboxyl group, a methoxy group, a peroxide group, and a polar ether group.

A coating layer for preventing non-specific adsorption and immobilizing a material to be immobilized on the substrate can be established on the substrate. By immobilizing the material on the substrate via such a coating layer, non-specific adsorption can be prevented and detection sensitivity can be enhanced. Furthermore, the amount of the material to be immobilized exposed on the uppermost surface is increased according to the method of immobilizing the material to be immobilized on the substrate via a coating layer, in comparison to the method comprising mixing a polymer, a material to be immobilized and a photocrosslinking agent, and then applying the mixture onto the substrate. Accordingly, a biochip substrate that is excellent in terms of S/N ratio and detection sensitivity can be obtained according to the method of immobilizing the material to be immobilized on the substrate via a coating layer.

The above-described coating layer is not particularly limited, as long as it enables promotion of the immobilization of a material to be immobilized, and/or suppression of non-specific adsorption. The coating layer can be constituted, for example, with various polymers. Among such polymers, a water-soluble polymer is preferable. By using such a water-soluble polymer, the use of water or a non-aqueous solvent other than alcohol, which may degenerate the material to be immobilized, can be avoided. Hence, in the present invention, in order to prevent degeneration of the material to be immobilized, a water-soluble polymer is preferably used. Moreover, the water-soluble polymer is advantageous in that it has an excellent effect of suppressing non-specific adsorption. Herein, the term "water-soluble" is used to mean that, for example, the solubility of the polymer in water (grams of the polymer dissolved in 100 g of water) is 5 or more.

The number average molecular weight of the above-described polymer is not particularly limited, and it is generally approximately 3,500,000 to 5,000,000. By setting the molecular weight of the polymer to be approximately 500 to several hundreds of thousands, the number of crosslinks between polymers can be moderately maintained, and the reaction of the material to be immobilized with a substance to be subjected to the reaction with the material to be immobilized (e.g., a photocrosslinking agent, etc.) can be promoted.

Examples of the water-soluble polymer may include bipolar polymers such as phosphorylcholine-containing polymers, and nonionic polymers. Examples of the nonionic polymers may include: polyalkylene glycol, such as polyethylene glycol (PEG) or polypropylene glycol; nonionic vinyl polymers comprising, as a constituent element(s), a single form of, or a mixture of monomer units, such as vinyl alcohol, methyl vinyl ether, vinyl pyrrolidone, vinyl oxazolidone, vinyl methyl oxazolidone, 2-vinyl pyridine, 4-vinyl pyridine, N-vinyl succinimide, N-vinyl formamide, N-vinyl-N-methyl formamide, N-vinyl acetamide, N-vinyl-N-methyl acetamide, 2-hydroxyethyl methacrylate, polyethylene glycol methacrylate, polyethylene glycol acrylate, acrylamide, methacrylamide, N,N-dimethyl acrylamide, N-isopropyl acrylamide, diacetone acrylamide, methylol acrylamide, acryloyl morpholine, acryloyl pyrrolidine, acryloyl piperidine, styrene, chloromethyl styrene, bromomethyl styrene, vinyl acetate, methyl methacrylate, butyl acrylate, methylcyano acrylate, ethylcyano acrylate, n-propylcyano acrylate, isopropylcyano acrylate, n-butylcyano acrylate, isobutylcyano acrylate, tert-butylcyano acrylate, glycidyl methacrylate, ethylvinyl ether, n-propylvinyl ether, isopropylvinyl ether, n-butylvinyl ether, isobutylvinyl ether, or tert-butylvinyl ether; and natural polymers, such as gelatin, casein, collagen, gum Arabic, xanthan gum, tragacanth gum, guar gum, pullulan, pectin, sodium alginate, hyaluronic acid, chitosan, a chitin derivative, carrageenan, starches (carboxymethyl starch and aldehyde starch), dextrin, or cyclodextrin, and natural polymers including water-soluble cellulose derivatives, such as methyl cellulose, viscose, hydroxyethyl cellulose, hydroxyethylmethyl cellulose, carboxymethyl cellulose, or hydroxypropyl cellulose, but the examples of the water-soluble polymer are not limited thereto. Moreover, commercially available products of photocrosslinked-type water-soluble polymers formed on the basis of these polymers, for example, "BIOSURFINE-AWP" manufactured by Toyo Gosei Co., Ltd., which was produced on the basis of polyvinyl alcohol, can be used. Among these polymers, polyethylene glycol polymers are particularly preferable, and among others, a vinyl polymer of polyethylene glycol (meth)acrylate is further particularly preferable.

In order to enhance the adhesiveness between the coating layer and the substrate, a surface treatment can be performed on the substrate. The surface treatment is not particularly limited, and examples of the surface treatment may include the treatments of cleaving the chemical bonds of molecules on the resin surface of the substrate and generating hydrophilic functional groups such as OH (hydroxyl groups), CO (carbonyl groups) and COOH (carboxyl groups), methoxy groups, peroxide groups, or polar ether group depending on the type of the resin, including, for example, a plasma treatment, a UV-ozone treatment, a corona treatment, or a flame treatment.

The biochip of the present invention is composed of, at least, the above-described substrate and a material to be immobilized, and has a reaction area for the biochemical reaction of a test sample on the upper surface of the substrate. The material to be immobilized comprises a biological material, and examples of such a biological material may include a peptide, a nucleic acid, a sugar chain, or a mixture of one or more types selected from these. It is to be noted that the term "peptide" is used herein as a generic name of substances formed by the peptide bonds of two or more amino acids. The peptide includes an oligopeptide and a polypeptide, and the polypeptide includes a protein.

The above-described biological material is not particularly limited. Preferred examples of the biological material may include: food allergens having immunogenicity, such as eggs, cow milks, meats, fish, crustaceans, mollusks, grains, beans, nuts, fruits, vegetables, beer yeasts, and gelatin; non-food allergens, such as pollens, mites, and house dusts; and tumor markers for liver cancer and breast cancer. Among others, specific examples of the biological material may include: main components of cow milk allergens, such as αs1-casein, αs2-casein, β-casein, κ-casein, α-lactalbumin, or β-lactoglobulin; main components of egg white allergens, such as ovomucoid, ovalbumin, or conalbumin; gliadin as a main component of wheat allergen; main proteins of *soba*, namely, proteins having a molecular weight of 24 kDa and 76 kDa; and Ara h1 as a main protein of peanuts. In particular, it is desirable to use at least one type of allergen selected from casein sodium, α-casein, β-casein, κ-casein, α-lactalbumin, β-lactoglobulin, ovomucoid, ovalbumin, and conalbumin. The above-described biological material may also include antibodies reacting against various types of allergens, biological materials (antigens, antibodies, aptamers, lectins, polynucleotides, enzymes, substrates, etc.) reacting with various types of biomarkers (e.g., a tumor marker, an infection marker, a genetic disease marker, an endocrine disease marker, an inflammation marker, a fatigue marker, a stress marker, and a nutrition marker), and antigens regarding pathogens, autoimmune diseases, etc. The biological material to be immobilized may be immobilized on an immobilized carrier which is immobilized on the reaction area.

The above-described immobilization carrier includes microparticle carriers such as organic microparticles and inorganic microparticles. The microparticle carriers may also be magnetic microparticles. Examples of the magnetic microparticles may include, but are not limited to, beads having a uniform particle diameter, in which magnetizable materials such as γFe₂O₃ and Fe₃O₄ are uniformly covered with polymers having hydrophilicity (e.g., water-soluble polymers such as glycidyl methacrylate). Examples of such beads as commercially available products may include Dynabeads manufactured by Thermo Fisher Scientific, FG Beads manufactured by TAMAGAWA SEIKI Co., Ltd., Sera-Mag Magnetic Beads manufactured by GE HealthCare, and Magnosphere manufactured by JSR Life Sciences.

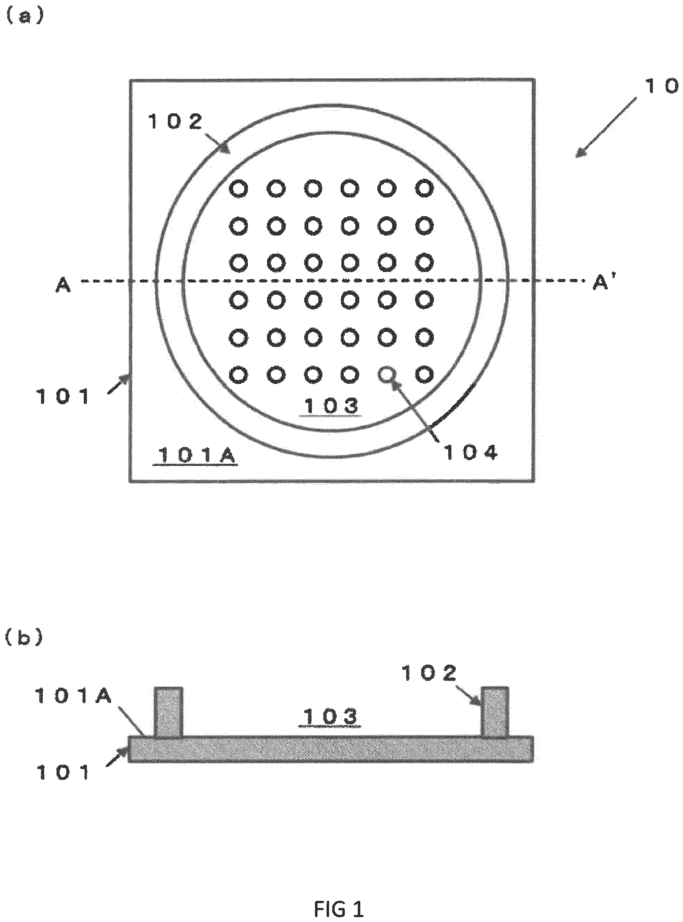

Figure 1(a) is a plan view showing one aspect of the biochip of the present invention (hereinafter also referred to as "Biochip (A)"). In Biochip (A) 10, ring-shaped Boundary 102 is formed on Upper Surface 101A of Substrate 101. The above-described Boundary 102 is formed into an annular shape having a predetermined width, and is protruded from Substrate 101 to a predetermined height, so as to form Reaction Area 103 for retaining a liquid in a space surrounded by Upper Surface 101A and Boundary 102 of Substrate 101. In Figure 1(a), a ring-shaped boundary is shown, but the shape of the boundary is not limited thereto, and various shapes such as oval and polygonal shapes (e.g., quadrangle, pentagon, hexagon, and octagon) may be adopted. In the above-described Reaction Area 103, Spot 104 on which a component reacting with the above-described test sample component (i.e., a material to be immobilized) is immobilized is arranged. A plurality of the above-described Spots 104 can be arranged. A plurality of independent spots are arranged at predetermined positions with predetermined intervals.

Figure 1(b) is a cross-sectional view showing a cross-sectional structure obtained by cutting Biochip (A) 10 with the line A-A' (see Figure 1(a)). The lower limit of the height of Boundary 102 from Upper Surface 101A of Substrate 101 is not particularly limited, but it is preferably 1 mm or more, and more preferably 2 mm or more. The upper limit of the height of Boundary 102 from Upper Surface 101A of Substrate 101 is not particularly limited, either. Taking into consideration the intended use of the chip, it is preferably 10 mm or less. The width of Boundary 102 is not particularly, either. Taking into consideration structural stability and the size of the chip, it is preferably in the range of 0.5 mm to 5.0 mm, and more preferably in the range of 1.0 mm to 3.0 mm. The material of Boundary 102 is not particularly limited, as long as it does not affect the test sample or the biochemical reaction. The material can be appropriately selected from rubber compositions such as natural rubber, synthetic rubber, silicon rubber, fluorine rubber or urethane rubber, or resin materials that can be used as the above-described substrate.

On Spot 104, the above-described material to be immobilized, or an immobilization carrier, on which the material to be immobilized has been immobilized, is immobilized. The number of Spots 104 or the arrangement thereof is not particularly limited, and any given number or arrangement can be applied. In terms of the simultaneous test of multiple items, the lower limit of the number of Spots 104 is preferably 3 or more, more preferably 12 or more, and particularly preferably 18 or more. From the viewpoint of the limited size of the reaction area and prevention of the overlapping of the detected signals with the signals of neighborhood spots upon detection, the upper limit of the number of Spots 104 is preferably 168 or less, and more preferably 144 or less.

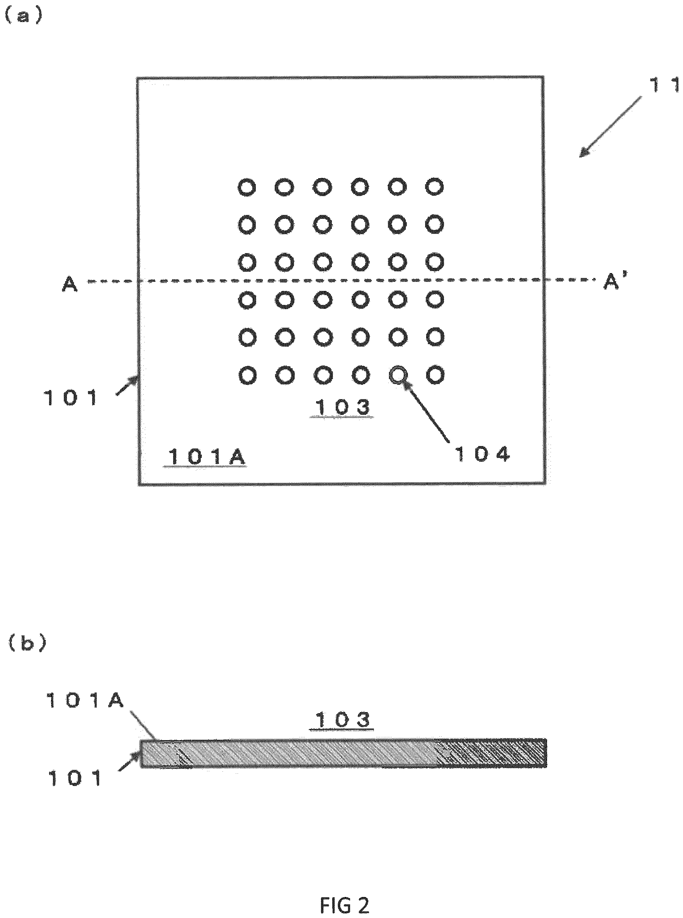

Figure 2(a) is a plan view showing another aspect of the biochip of the present invention (hereinafter also referred to as "Biochip (B)"). Biochip (B) 11 is different from Biochip (A) in that it does not have a boundary and the entire Upper Surface 101A of Substrate 101 serves as Reaction Area 103, but Biochip (B) 11 is identical to Biochip (A) in terms of other configurations. Since Biochip (B) does not need to establish a boundary, it is advantageous in terms of a simple production process and low production costs.

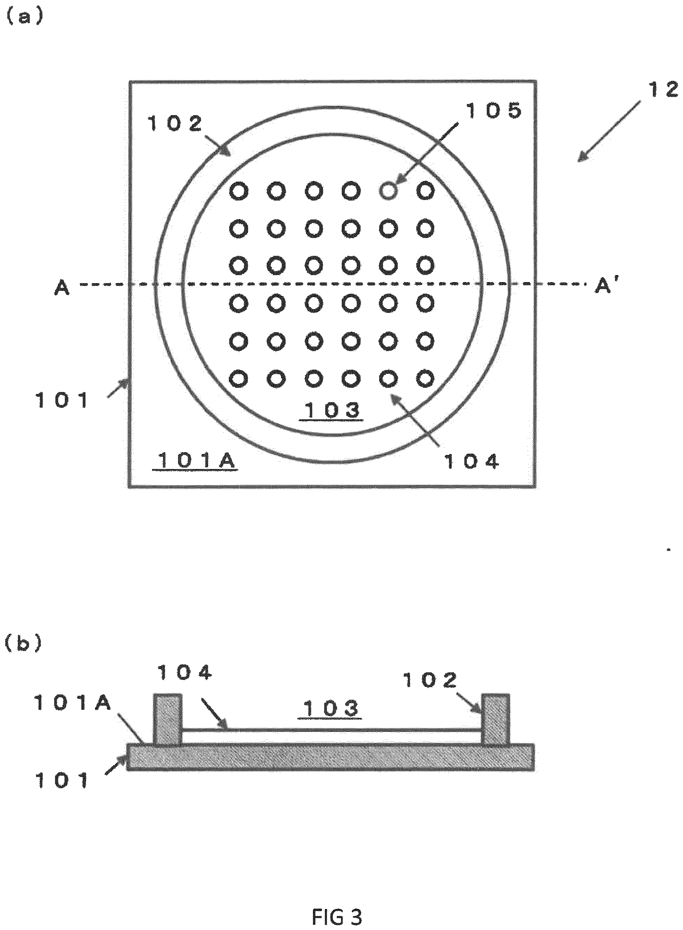

Figure 3(a) is a plan view showing one aspect of the biochip of the present invention (hereinafter also referred to as "Biochip (C)"). In Biochip (C) 12, ring-shaped Boundary 102 is formed on Upper Surface 101A of Substrate 101. The above-described Boundary 102 is formed into an annular shape having a predetermined width, and is protruded from Substrate 101 to a predetermined height, so as to form Reaction Area 103 for retaining a liquid in a space surrounded by Upper Surface 101A and Boundary 102 of Substrate 101. In Figure 3(a), a ring-shaped boundary is shown, but the shape of the boundary is not limited thereto, and various shapes such as oval and polygonal shapes (e.g., quadrangle, pentagon, hexagon, and octagon) may be adopted. In the above-described Reaction Area 103, Coating Layer 104 is formed to prevent non-specific adsorption and to immobilize biological materials on the substrate, and a plurality of Spots 105, on which components reacting with the above-described test sample components (i.e., materials to be immobilized) have been immobilized, are arranged on the substrate via the Coating Layer 104. The above-described Spots 105 are arranged as a plurality of independent spots at predetermined positions with predetermined intervals.

Figure 3(b) is a cross-sectional view showing a cross-sectional structure obtained by cutting Biochip (C) 12 with the line A-A' (see Figure 3(a)). The lower limit of the height of Boundary 102 from Upper Surface 101A of Substrate 101 is not particularly limited, but it is preferably 1 mm or more, and more preferably 2 mm or more. The upper limit of the height of Boundary 102 from Upper Surface 101A of Substrate 101 is not particularly limited, either. Taking into consideration the intended use of the chip, it is preferably 10 mm or less. The width of Boundary 102 is not particularly, either. Taking into consideration structural stability and the size of the chip, it is preferably in the range of 0.5 mm to 5.0 mm, and more preferably in the range of 1.0 mm to 3.0 mm. The material of Boundary 102 is not particularly limited, as long as it does not affect the test sample or the biochemical reaction. The material can be appropriately selected from rubber compositions such as natural rubber, synthetic rubber, silicon rubber, fluorine rubber, or urethane rubber, or resin materials that can be used as the above-described substrate.

The thickness of Coating Layer 104 is not particularly limited, and any given thickness can be formed. The thickness is preferably 1 nm to 10 µm, more preferably 2 nm to 1 µm, and particularly preferably 10 nm to 100 nm.

On Spot 105, the above-described material to be immobilized, or an immobilization carrier, on which the material to be immobilized has been immobilized, is immobilized. The number of Spots 105 or the arrangement thereof is not particularly limited, and any given number or arrangement can be applied. In terms of the simultaneous test of multiple items, the lower limit of the number of Spots 105 is preferably 3 or more, more preferably 12 or more, and particularly preferably 18 or more. From the viewpoint of the limited size of the reaction area and prevention of the overlapping of the detected signals with the signals of neighborhood spots upon detection, the upper limit of the number of Spots 105 is preferably 168 or less, and more preferably 144 or less.

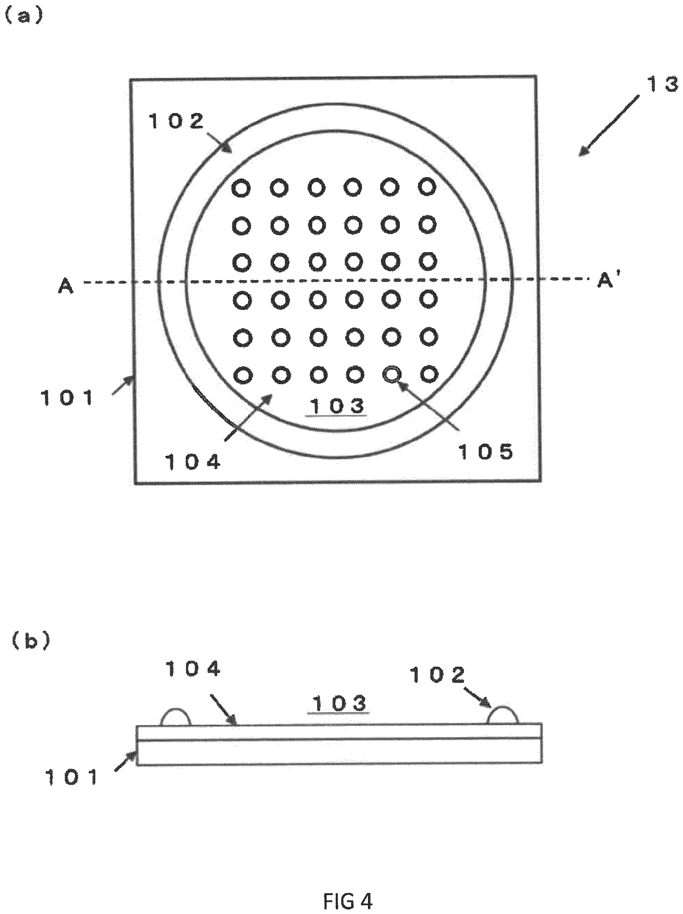

Figure 4(a) is a plan view showing another aspect of the biochip of the present invention (hereinafter also referred to as "Biochip (D)"). Biochip (D) 13 is different from Biochip (C) in that Coating Layer 104 for preventing non-specific adsorption and immobilizing biological materials is formed on the entire upper surface of Substrate 101. Accordingly, ring-shaped Boundary 102 is formed on Coating Layer 104. The above-described Boundary 102 is formed into an annular shape having a predetermined width, and is protruded from Coating Layer 104 to a predetermined height, so as to form Reaction Area 103 for retaining a liquid in a space surrounded by Coating Layer 104 and Boundary 102. The cross-section of Boundary 102 in this aspect is a semi-cylindrical form. However, the cross-sectional shape of Boundary 102 is not particularly limited, as long as a liquid can be retained in the space surrounded by Boundary 102. Thus, various shapes such as a triangle, a trapezoidal shape, or an L-shape can be adopted, as well as a rectangle as in the case of Biochip A. A plurality of Spots 105, on which components reacting with the above-described test sample components (i.e., materials to be immobilized) have been immobilized, are arranged on Coating Layer 104 of the above-described Reaction Area 103. The above-described Spots 105 are arranged as a plurality of independent spots at predetermined positions with predetermined intervals.

Figure 4(b) is a cross-sectional view showing a cross-sectional structure obtained by cutting Biochip (D) 13 with the line A-A' (see Figure 4(a)). The range of the height of Boundary 102 from Coating Layer 104 is the same as the range of the height of Boundary 102 from Upper Surface 101A of Substrate 101 in Biochip (C). Also, the width and material of Boundary 102, the thickness of Coating Layer 104, and Spots 105 are the same as those of Biochip (C).

### Method for producing biochip of the present invention

The method for producing the biochip of the present invention comprising a hydrophilic reaction area may comprise:
producing a biochip substrate by a step of performing a hydrophilization treatment on a substrate (hereinafter referred to as "Step 1-1"); and then,
a step of spotting, at least, a material to be immobilized, a photocrosslinking agent having at least two photoreactive groups in a single molecule, and a thickener and a surfactant on the above-described biochip substrate, and then applying a light to the substrate (hereinafter referred to as "Step 1-2").

Hereafter, individual constituent elements of the production method of the present invention will be described, successively.

### Step 1-1

Step 1-1 may comprise a step of performing a hydrophilization treatment on a substrate. The hydrophilization treatment of the substrate is not particularly limited, as long as it is a treatment of hydrophilizing the surface of the substrate. Examples of the hydrophilization treatment may include: a method comprising applying silica, a surfactant and the like that are dissolved or suspended in a liquid onto a substrate according to spin-coating, coating, spraying, immersion, etc., and then drying it; a method of performing a chemical hydrophilization treatment such as a plasma treatment, a UV-ozone treatment, a corona treatment, or a flame treatment on a substrate; a method of transcribing a fine nanostructure on a substrate, or roughening a substrate with a liquid medicine to physically form a nanostructure on the substrate, so as to impart hydrophilicity to the substrate; and a method of coating a substrate with a hydrophilic polymer. A method of performing a chemical hydrophilization treatment such as a plasma treatment, a UV-ozone treatment, a corona treatment, or a flame treatment on a substrate, in which the chemical bonds of molecules on the resin surface are cleaved and thereby hydrophilic functional groups having polarity, such as OH (hydroxyl groups), CO (carbonyl groups), COOH (carboxyl groups), methoxy groups, peroxide groups, or polar ether groups can be generated depending on the type of the resin, is more preferable, and a method involving a UV-ozone treatment is particularly preferable.

A step of forming a boundary on a substrate to form a reaction area can be established. The method of forming a reaction area is not particularly limited. Examples of the method of forming a reaction area that can be used herein may include: a method of allowing a ring that has previously been formed with the above-described rubber composition or the above-described resin material to adhere to a substrate, using an adhesive or the like; and a method of subjecting a substrate constituted with the above-described resin material to various types of resin molding methods such as injection molding or vacuum forming, or to mechanical cutting or the like, so as to mold a ring. The adhesive used herein is not particularly limited, as long as it does not affect the test sample or the biochemical reaction. The adhesive can be appropriately selected from commercially available adhesives.

### Step 1-2

Step 1-2 may comprise a step of spotting, at least, a material to be immobilized, a photocrosslinking agent having at least two photoreactive groups in a single molecule, and a thickener and a surfactant, in the form of grid-like spots, on above-described biochip substrate, and a step of applying a light to the substrate.

The method for producing the biochip of the present invention having a coating layer and a reaction area surrounded by a boundary capable of retaining a liquid therein may comprise:
producing a biochip substrate by a step selected from a step of establishing a coating layer for preventing non-specific adsorption and immobilizing biological materials on the substrate, and a step of forming a boundary on the substrate to form a reaction area (hereinafter referred to as "Step 2-1"); and then,
a step of spotting, at least, a material to be immobilized, a photocrosslinking agent having at least two photoreactive groups in a single molecule, and a thickener and a surfactant on the above-described biochip substrate, and then applying a light to the substrate (hereinafter referred to as "Step 2-2").

Hereafter, individual constituent elements of the method for producing the biochip of the present invention having a coating layer and a reaction area surrounded by a boundary capable of retaining a liquid therein will be described, successively.

### Step 2-1

Step 2-1 may comprise a step selected from a step of establishing a coating layer for preventing non-specific adsorption and immobilizing biological materials on the substrate and a step of forming a boundary on the substrate to form a reaction area. As long as Step 2-1 comprises one or more steps selected from the above-described steps, the order of performing these steps may be changed. In addition, Step 2-1 may comprise a step of performing a surface treatment on the substrate, before the step of establishing a coating layer.

The method of performing a surface treatment in the step of performing a surface treatment on the substrate is not particularly limited, as long as the method can enhance the adhesiveness between the substrate and the coating layer. For example, a known surface modification method such as a plasma treatment, a UV-ozone treatment, or a corona treatment can be applied.

A coating layer for preventing non-specific adsorption on a substrate and immobilizing biological materials on the substrate can be formed according to a known method such as the spin-coating, coating or spraying of a coating solution comprising a water-soluble polymer, or immersion of the substrate in a coating solution. For example, the coating solution can be prepared by dissolving a water-soluble polymer in a solvent. As such solvents, water, a lower alcohol that is mixed with water at any given ratio, and a mixture thereof can be used. As such lower alcohols, methanol, ethanol, and isopropanol are preferable. Among others, it is preferable to use a mixed solvent of ethanol and water.

The concentration of the polymer in the above-described coating solution is not particularly limited. The polymer concentration can be set at, for example, 0.0001 to 10 parts by mass, and preferably 0.001 to 1 part by mass. The concentration of the photocrosslinking agent can be set at, for example, 1 to 20 parts by mass, and preferably 2 to 10 parts by mass, with respect to the above-described polymer.

The coating solution containing the above-described polymer preferably comprises a photocrosslinking agent having at least two photoreactive groups in a single molecule. In the present invention, the "photoreactive group" means a group that generates radicals as a result of light irradiation.

In the above-described photocrosslinking agent, photoreactive groups generate radicals as a result of light irradiation, so that covalent bonds can be formed with amino groups, carboxyl groups, carbon atoms constituting an organic compound, etc. Thereby, a coating solution comprising the above-described photocrosslinking agent is applied onto the substrate, followed by light irradiation, so that the substrate can be bound to the polymer via the photocrosslinking agent, and so that a polymer layer having a non-specific adsorption preventing effect can be formed on the substrate. Besides, in the present invention, without using the above-described photocrosslinking agent, or together with the above-described photocrosslinking agent, a photoreactive group and/or a group capable of covalently or coordinately binding to the substrate surface are introduced into the above-described polymer, so that the substrate can be bound to the polymer by utilizing the groups possessed by the polymer.

The water-soluble polymer and the photocrosslinking agent comprised in the coating layer are known. The water-soluble polymer and the photocrosslinking agent can be produced by known production methods and also, are commercially available. The film thickness of the above-described coating layer is not particularly limited, but it is preferably 1 nm to 10 µm, more preferably 2 nm to 1 µm, and particularly preferably 10 nm to 100 nm.

In the present invention, as mentioned above, it is preferable to stabilize a coating layer by applying the coating layer to the substrate and then aging it under constant temperature and humidity conditions. The temperature is preferably 5°C to 40°C, and more preferably 20°C to 30°C. The humidity is preferably 40% to 80%, and more preferably 50% to 70%. The aging period is preferably 1 day to 1 month, and more preferably 3 days to 2 weeks.

The method of forming a boundary on the substrate is not particularly limited. A method of adhering a ring that has previously been formed with the above-described rubber composition or the above-described resin material onto the substrate, using an adhesive or the like, or a method of molding a substrate constituted with the above-described resin material into a ring according to various types of resin molding methods such as injection molding or vacuum forming, mechanical cutting, etc., can be applied. The adhesive is not particularly limited, as long as it does not affect the test sample or the biochemical reaction. Thus, an adhesive can be appropriately selected from commercially available adhesives.

### Step 2-2

Step 2-2 may comprise: a step of spotting, at least, a material to be immobilized, a photocrosslinking agent having at least two photoreactive groups in a single molecule, and a thickener and a surfactant, in the form of grid-like spots, on the above-described biochip substrate; a step of applying a light to the substrate; and a step of removing unreacted components.

In Step 1-2 or 2-2, when the above-described material to be immobilized has previously been immobilized on an immobilization carrier, a known immobilization method can be applied. The immobilization method may be, for example, a method which comprises allowing the above-described magnetic microparticles used as immobilization carriers to react with the previously prepared materials to be immobilized in a solution such as an appropriately selected known buffer, and then recovering the magnetic microparticles on which the materials to be immobilized have been immobilized, but the immobilization method is not limited thereto. For example, the above-described magnetic microparticles may have previously been washed with a known buffer, or when the magnetic microparticles on which the materials to be immobilized have been immobilized are recovered, the recovered magnetic microparticles may be washed with a known buffer.

The photocrosslinking agent having at least two photoreactive groups in a single molecule, which is used in Step 1-2 or 2-2, is not particularly limited. Examples of the photoreactive groups possessed by the above-described photocrosslinking agent may include azide groups (-N₃), acetyl groups, benzoyl groups and diazirine groups. In particular, azide groups are preferable because when the azide groups are irradiated with light, nitrogen molecules are dissociated and nitrogen radicals are generated, and these nitrogen radicals can bind not only to functional groups such as amino groups or carboxyl groups, but can also bind to carbon atoms constituting an organic compound, and can form covalent bonds with almost all organic matters. The photocrosslinking agent having such azide groups may be, for example, diazidostilbene.

The photocrosslinking agent is preferably a water-soluble agent. The "water solubility" of the photocrosslinking agent means that an aqueous solution comprising the photocrosslinking agent in a concentration of 0.5 mM or more, and preferably 2 mM or more, can be given.

The above-described material to be immobilized, or an immobilization carrier on which the material to be immobilized has been immobilized, and the above-described photocrosslinking agent are preferably dispersed or dissolved in a solution. The solution is not particularly limited, and a known buffer can be used. Examples of the buffer composition may include a PBS buffer, a HEPES buffer, a Tris buffer, and a MES buffer. When the material to be immobilized is directly used, a phosphate buffered saline is preferably used. When the material to be immobilized is immobilized on an immobilization carrier and is then used, a HEPES buffer is preferably used to prevent agglutination of the immobilization carrier. The solution, in which the material to be immobilized and the photocrosslinking agent are dispersed or dissolved, may be referred to as a stamp solution at times.

The concentration of the above-described material to be immobilized is not particularly limited. When the material to be immobilized is not immobilized on an immobilization carrier, the concentration of the material to be immobilized is preferably 0.05 mg/mL to 2 mg/mL, and more preferably 0.1 mg/mL to 1 mg/mL. When the material to be immobilized is immobilized on an immobilization carrier, the material to be immobilized is allowed to react with the immobilization carrier in the concentration of the material to be immobilized that is preferably 0.5 mg/mL to 50 mg/mL, and more preferably 1 mg/mL to 25 mg/mL. The immobilization carrier, on which the material to be immobilized has been immobilized, is used in a concentration of preferably 1 mg/mL to 10 mg/mL, and more preferably 2.5 mg/mL to 7.5 mg/mL.

The concentration of the above-described photocrosslinking agent is not particularly limited, but it is preferably 0.01 mg/mL to 1 g/mL, and more preferably 0.2 mg/mL to 0.2 g/mL.

The solution, in which the above-described material to be immobilized, or the immobilization carrier on which the material to be immobilized has been immobilized, and the above-described photocrosslinking agent are dispersed or dissolved, further comprises a thickener and a surfactant. By allowing the above-described solution to comprise a thickener, the size of a spot can be controlled when the solution is spotted on a biochip substrate. More specifically, when a solution having a predetermined volume is spotted on a substrate, as the concentration of a thickener in the solution increases, the size of a spot (the area of a spot contacted with the substrate) tends to decrease. Moreover, by allowing the solution to comprise a surfactant, a phenomenon, whereby the material to be immobilized accumulates in the gas-liquid interface and the material to be immobilized is thereby localized in the margin of the spot, can be prevented. Also, such a surfactant contributes to the improvement of the affinity for the substrate. When a solution having a predetermined volume is spotted on a substrate, as the concentration of a surfactant in the solution increases, the size of a spot (the area of a spot contacted with the substrate) tends to increase. Accordingly, a stamp with any given size can be formed by controlling the concentration of the thickener and the surfactant.

The above-described thickener is gellan gum, xanthan gum, curdlan, pullulan, a guar gum derivative, locust bean gum, carrageenan, pectin, tamarind gum, psyllium seed gum, dextran, glycerin, carboxymethyl cellulose, hydroxyethyl cellulose, hydroxymethylpropyl cellulose, lanolin, methyl cellulose, Vaseline, polyethylene glycol, polyvinyl alcohol, polyvinyl pyrrolidone, a carboxyl vinyl polymer, polyvinyl pyrrolidone, polyvinyl alcohol, dextrin acid fatty ester, inulin acid fatty ester, or a mixture of two or more types selected from these. It is preferable to use one or more selected from hydroxyethyl cellulose, methyl cellulose, polyvinyl pyrrolidone, and polyvinyl alcohol, and it is more preferable to use polyvinyl alcohol.

The concentration of the above-described thickener is not particularly limited. For example, when polyvinyl alcohol is used as a thickener, it is used in a concentration of preferably 0.01 part by weight to 1 part by weight, more preferably 0.02 parts by weight to 0.5 parts by weight, and particularly preferably 0.03 parts by weight to 0.3 parts by weight, with respect to 100 parts by weight of the above-described solution.

The above-described surfactant is a nonionic surfactant. Examples of the surfactant that can be used herein may include polyoxyethylene(10) octylphenyl ether [Triton X-100], polyoxyethylene(8) octylphenyl ether [Triton X-114], polyoxyethylene sorbitan monolaurate [Tween 20], polyoxyethylene sorbitan monooleate [Tween 80], polyoxyethylene(23) lauryl ether [Brij35], polyoxyethylene(20) lauryl ether [Brij58], Pluronic F-68, polyethylene glycol, and a mixture consisting of two or more types thereof. It is preferable to use one or more types selected from Triton X-100, Tween 20, and Tween 80, and it is more preferable to use Tween 20.

The concentration of the above-described surfactant is not particularly limited. For example, when Tween 20 is used as a surfactant, it is used in a concentration of preferably 0.001 part by weight to 1 part by weight, more preferably 0.005 parts by weight to 0.5 parts by weight, and particularly preferably 0.01 part by weight to 0.3 parts by weight, with respect to 100 parts by weight of the above-described solution.

The viscosity of the stamp solution is not particularly limited, as long as a spot having a desired shape can be formed. The viscosity of the stamp solution at 25°C may be, for example, 0.4 mPa-s to 40 mPa·s, preferably 0.5 mPa-s to 10 mPa·s, more preferably 0.6 mPa-s to 4 mPa·s, particularly preferably 0.8 mPa-s to 2 mPa·s, and further particularly, approximately 1.3 mPa·s. On the other hand, the viscosity of the stamp solution at 25°C may be 0.6 mPa-s to 10 mPa·s, 0.8 mPa-s to 4 mPa·s, 1.0 mPa-s to 2 mPa·s, 1.12 mPa-s to 2 mPa·s, 1.13 mPa-s to 2 mPa·s, 1.14 mPa·s to 2 mPa·s, 1.15 mPa·s to 2 mPa·s, 1.16 mPa·s to 2 mPa·s, 1.17 mPa-s to 2 mPa·s, 1.18 mPa-s to 2 mPa·s, 1.19 mPa-s to 2 mPa·s, 1.2 mPa-s to 2 mPa·s, or the like.

The method of spotting a material to be immobilized or an immobilization carrier on which a material to be immobilized has been immobilized, and a photocrosslinking agent, onto the above-described biochip substrate, is not particularly limited. For example, a method of spotting them using a micropipette or the like, a spotting method involving a pin method, a spotting method involving a piezoelectric method, etc. can be applied. Among these, a spotting method of using a micropipette or the like and a spotting method involving a pin method, which are not restricted by the size of a microparticle, are preferable. The diameter of such a spot can be set at, for example, 400 µm to 800 µm, and preferably 500 µm to 700 µm.

Immobilization of a material to be immobilized, or an immobilization carrier on which a material to be immobilized has been immobilized, can be carried out by applying a light to a substrate, after application of a solution onto the substrate, preferably after the drying of the applied solution. The light is not particularly limited, as long as the used photoreactive groups can generate radicals under the light. In particular, when azide groups are used as such photoreactive groups, ultraviolet rays (for example, with a wavelength of 10 to 400 nm) are preferable. The irradiation time can be set at, for example, 10 seconds to 120 minutes, preferably 30 seconds to 60 minutes, and more preferably 1 minute to 30 minutes. Since the material to be immobilized is promptly immobilized by light irradiation, the irradiation time is almost equal to the time required for immobilization. The dose of the irradiated light is not particularly limited, but it is generally approximately 1 mW to 100 mW per cm². The photoreactive groups contained in the photocrosslinking agent (or when the polymer has photoreactive groups, the photoreactive groups contained in the polymer) generate radicals as a result of the light irradiation. When the material to be immobilized is not immobilized on the immobilization carrier, the polymer layer can be bound to the material to be immobilized via the photocrosslinking agent. When the material to be immobilized is immobilized on the immobilization carrier, the polymer layer can be bound to the material to be immobilized and/or the immobilization carrier via the photocrosslinking agent.

In the present invention, after a desired material has been immobilized on a substrate as described above, the substrate is washed according to a known method, so that unreacted components and the like can be removed. However, such washing is not essential, and the immobilized material may be subjected to productization, while the stamp solution remains. Thus, a biochip, on which a desired material to be immobilized has been immobilized, can be obtained. When the washing is not carried out, the immobilized material, as well as stamp solution components such as a thickener and/or a surfactant, may adhere onto the spot on the biochip. Such a stamp solution residue can be removed by washing the biochip, as appropriate, before the use thereof.

### Method for preserving biochip

A deoxygenation means may be performed on a material to be immobilized, which is affected by oxidation, in particular, amino acids in a protein, etc., so as to obtain the effect of preventing the oxidation of the material to be immobilized and/or the effect of suppressing the proliferation of aerobic bacteria and fungi on the biochip.

Examples of the deoxygenation means may include, but are not limited to, the packaging of the biochip by at least one form of the following (a) to (d): (a) vacuum packaging; (b) packaging together with inert gas; (c) packaging together with an oxygen scavenger; and (d) packaging with a wrapping material having deoxygenation function. Accordingly, examples of the deoxygenation means may also include a combination of (a) and (c) (i.e., vacuum packaging together with an oxygen scavenger), a combination of (b) and (c) (i.e., packaging together with inert gas and an oxygen scavenger), and a combination of (b) to (d) (i.e., packaging with a wrapping material having deoxygenation function, together with inert gas and an oxygen scavenger), in addition to each of the above packaging forms (a) to (d).

Specific examples of such a deoxygenation means may include a method of performing the replacement of inert gas such as nitrogen or argon upon the aforementioned packaging of a biochip, a method of enclosing an oxygen scavenger in a package, and a method of using a wrapping material having deoxygenation function, for example, a wrapping material film.

Examples of the oxygen scavenger allowed to coexist in the package may include: inorganic oxygen scavengers such as iron or cerium oxide; and organic oxygen scavengers. Such an oxygen scavenger is not particularly limited, and specific examples may include commercially available oxygen scavengers that can be used for medical purposes, such as "AGELESS" manufactured by MITSUBISHI GAS CHEMICAL COMPANY, INC., and "PharmaKeep" manufactured by MITSUBISHI GAS CHEMICAL COMPANY, INC.

The wrapping material having deoxygenation function is not particularly limited, and a wrapping film containing an oxygen scavenger is preferable. Such a wrapping film can be specifically classified into wrapping films containing an inorganic oxygen scavenger such as iron or cerium oxide, and wrapping films containing an organic oxygen scavenger. These films are not particularly limited. Examples of the wrapping film may include commercially available films that can be used for medical purposes, for example, wrapping materials for wrapping cerium oxide, such as "OxyCatch" manufactured by Kyodo Printing Co., Ltd., and "High Star 02" manufactured by Starplastic Industry Inc. Examples of wrapping materials for wrapping ion that can be used herein may include "AGELESS OMAC" manufactured by MITSUBISHI GAS CHEMICAL COMPANY, INC., and "OxyGuard" manufactured by Toyo Seikan Co., Ltd.

Furthermore, even in a case where a biochip cannot sufficiently stably maintain a material to be immobilized thereon only by the deoxygenation means, the biochip of the present invention can stably maintain the material to be immobilized thereon by the combined use of a desiccant in some cases. In such a case, it is preferable to use a desiccant in combination. Naturally, it is also possible to allow a desiccant having only dehumidification function to coexist in the package, and then to prepare a package wrapped by a wrapping material having deoxygenation function. As such a desiccant, a desiccant that is commonly used in the field of pharmaceutical products can be used.

### Package that packages biochip of the present invention

The present invention also relates to a package that packages a biochip according to the above-described preservation method. The package of the present invention may package the biochip of the present invention by at least one form of the following (a) to (d): (a) vacuum packaging; (b) packaging together with inert gas; (c) packaging together with an oxygen scavenger; and (d) packaging with a wrapping material having deoxygenation function. Moreover, the package of the present invention may further enclose a desiccant. The above-described individual configurations regarding the package of the present invention are as described above regarding the preservation method. The package of the present invention can preserve the biochip of the present invention for a long period of time without impairing the quality of the biochip of the present invention, and thus, it is excellent in terms of practicality.

### Inspection of biological sample by using biochip

Further described herein are a method of testing a biological sample by using the above-described biochip, a method of testing the health condition of a subject, a method of testing or diagnosing a disease in a subject, the above-described biochip for use in such inspection and/or diagnostic application, and a kit for use in inspection and/or diagnostic application, comprising the above-described biochip.

The method of testing a biological sample by using the biochip of the present invention comprises:
(i) a step of providing the above-described biochip comprising a material reacting with a target substance as a material to be immobilized;
(ii) a step of allowing the above-described biochip to react with a biological sample; and
(iii) a step of detecting the reaction of the above-described material to be immobilized with a target substance contained in the biological sample, wherein detection of the above-described reaction indicates the presence of the target substance in the biological sample, and non-detection of the above-described reaction indicates the absence of the target substance in the biological sample.

Examples of the target substance may include, but are not limited to, proteins such as antibodies, antigens, enzymes, hormones or cytokines, nucleic acid molecules such as RNA or DNA, and sugar chains.

The material reacting with the target substance is not limited, and examples of the material may include antigens, antibodies, aptamers, lectins, polynucleotides, enzymes, and substrates.

The reaction of the material to be immobilized with the target substance can be detected by various types of known means. For example, when the target substance is a protein, the protein is allowed to react with a labeled antibody reacting against the protein, and then, the label of the labeled antibody binding to the protein can be detected by a method suitable for detection of the label. On the other hand, when the target substance is a nucleic acid molecule, the nucleic acid molecule is allowed to react with a labeled probe hybridizing with the nucleic acid molecule, and then, the label of the labeled probe binding to the nucleic acid molecule can be detected by a method suitable for detection of the label. Examples of the label may include, but are not limited to, a fluorescent material, a luminescent material, an enzyme, and a radioisotope. Examples of the method of detecting the label may include, but are not limited to, spectrophotometry, absorptiometry, fluorometry, colorimeteric method, and autoradiography. The detection may be either qualitative detection or quantitative detection. When quantitative detection is carried out, information regarding the concentration of a target substance in a biological sample, etc. can be obtained.

The method of testing the health condition of a subject by using the biochip of the present invention comprises:
(i) a step of providing the above-described biochip comprising a material reacting with a target substance as a material to be immobilized;
(ii) a step of allowing the above-described biochip to react with a biological sample derived from a subject; and
(iii) a step of detecting the reaction of the above-described material to be immobilized with a target substance contained in the biological sample, wherein detection or non-detection of the above-described reaction provides information regarding the health condition of the subject.

Examples of the information regarding health condition may include fatigue, stress, and nutritional status. Examples of the target substance may include biological materials regarding the information, such as proteins, nucleic acid molecules, or sugar chains. Examples of the material to be immobilized may include materials reacting with these biological materials, such as antigens, antibodies, aptamers, lectins, polynucleotides, enzymes, or substrates. Biomarkers used as indicators for fatigue, stress, nutritional status, etc. have been known.

The method of testing or diagnosing a disease in a subject by using the biochip of the present invention comprises:
(i) a step of providing the above-described biochip comprising a material reacting with a target substance as a material to be immobilized;
(ii) a step of allowing the above-described biochip to react with a biological sample derived from a subject; and
(iii) a step of detecting the reaction of the above-described material to be immobilized with a target substance contained in the biological sample, wherein detection or non-detection of the above-described reaction provides the presence or absence of the disease in the subject.

The disease may be a disease that can be tested and/or diagnosed by detecting a substance in the biological sample. Specific examples of the disease may include, but are not limited to, allergic disease, endocrine disease, infectious disease, disease with genetic abnormality, inflammatory disease, autoimmune disease, and neoplastic disease. Examples of the target substance may include biological materials regarding such diseases, such as proteins, nucleic acid molecules, or sugar chains. Examples of the material to be immobilized may include materials reacting with these biological materials, such as antigens, antibodies, aptamers, lectins, polynucleotides, enzymes, or substrates. Various types of disease markers such as a tumor marker, an infectious disease marker, a genetic disease marker, an endocrine disease marker, or an inflammation marker have been known.

The biochip of the present invention can be used in each of the above-described intended uses, namely, inspection of a biological sample, inspection of the health condition of a subject (e.g., fatigue, stress, nutritional status, etc.), and inspection or diagnosis of a disease in a subject (e.g., allergic disease, endocrine disease, infectious disease, disease with genetic abnormality, inflammatory disease, autoimmune disease, neoplastic disease, etc.). The biochip of the present invention for use in each of the above-described intended uses preferably comprises a material to be immobilized that is suitable for each of the above-described intended uses.

The kit can be used in each of the above-described intended uses, namely, inspection of a biological sample, inspection of the health condition of a subject (e.g., fatigue, stress, nutritional status, etc.), and inspection or diagnosis of a disease in a subject (e.g., allergic disease, endocrine disease, infectious disease, disease with genetic abnormality, inflammatory disease, autoimmune disease, neoplastic disease, etc.). The kit includes the biochip of the present invention, and preferably the biochip of the present invention comprising a material to be immobilized that is suitable for each of the above-described intended uses. The kit may include a reagent for detecting the reaction of the material to be immobilized with a target substance contained in a biological sample, a standard sample, instructions showing the method of using the kit, etc. such as, for example, an instruction manual, and a medium recording information regarding the use method, such as, for example, a flexible disc, CD, DVD, a Blu-ray disc, a memory card, or an USB memory, as well as the biochip of the present invention.

### Examples

Hereinafter, the present invention will be described in more detail in the following examples. However, these examples are not intended to limit the present invention.

### < Example 1: Production of biochip substrate (1) >

A polycarbonate sheet (manufactured by MITSUBISHI GAS CHEMICAL COMPANY, INC., MU58U, thickness: 0.8 mm) was irradiated with ultraviolet rays, using a UV-ozone irradiation device (manufactured by SEN LIGHTS Co., Ltd., SSP16-110, UV lamp: SUV110GS-36L) at an irradiation distance of 50 mm for 2 minutes. Subsequently, a polyethylene glycol monomethacrylate polymer (manufactured by Sanyu Chemical Co., Ltd.; the molecular weight of a polyethylene glycol moiety: 350) and 4,4'-diazidostilbene-2,2'-disulfonic acid (manufactured by TOKYO CHEMICAL INDUSTRY CO., LTD.; 98%; hereinafter referred to as "bisazide") were each dissolved in a 75% ethanol aqueous solution to result in 0.5 parts by weight and 0.025 parts by weight, respectively, thereby obtaining a coating solution of non-specific adsorption preventing agent. Using a spin-coater (manufactured by MIKASA, MS-A100), the above-described UV-irradiated polycarbonate was coated with 15 µL of this coating solution under conditions of 800 rpm for 5 seconds, and 5000 rpm for 10 seconds. After completion of the coating, using a UV irradiation device (manufactured by UVP, CL-1000), the substrate was irradiated with ultraviolet rays at 120 mW/cm² for 10 minutes. A silicon rubber-made O ring (14 ϕ) was adhered to the thus coated substrate, using an adhesive, so as to form a reaction area. The aging operation was performed at a temperature of 25°C at a humidity of 65% for 4 days, so as to obtain Biochip Substrate 1.

Biochip Substrate 2 was obtained in the same manner as that for Biochip Substrate 1, with the exception that a reaction area was not formed with a silicon rubber.

### < Example 2: Preparation of stamp solution >

### (1) (Stamp Solution 1-1)

Sodium chloride, potassium chloride, disodium hydrogen phosphate dodecahydrate, and potassium dihydrogen phosphate were each dissolved in ultrapure water to result in 0.8 parts by weight, 0.02 parts by weight, 0.29 parts by weight, and 0.02 parts by weight, respectively, so as to obtain a PBS solution. On the other hand, 4-(2-hy droxy ethyl)-1 -piperazineethanesulfonic acid was dissolved in ultrapure water to result in 0.59 parts by weight, so as to obtain a HEPES solution. Polyvinyl alcohol (manufactured by Wako Pure Chemical Industries, Ltd., 160-03055) and Tween 20 (manufactured by Sigma-Aldrich, P7949-100ML) were each dissolved in the HEPES solution to result in 0.1 part by weight and 0.05 parts by weight, respectively, so as to obtain a solvent for stamp solution. Biotinylated peptides formed by biotinylating the termini of partial peptides of αs1-casein (consisting of 15 amino acid residues), which were used as materials to be immobilized, were allowed to react with Streptavidin beads (manufactured by TAMAGAWA SEIKI Co., Ltd.) in the PBS solution at 4°C for 1.5 hours, were then purified, and were then dispersed in the solvent for stamp solution, so as to obtain Stamp Solution 1-1.

The viscosity of Stamp Solution 1-1 was measured. The measurement was carried out using B-type Viscometer DV2T (manufactured by Brookfield) under conditions of 20°C and 200 rpm. Before the measurement of a sample, correction was carried out using water and a standard liquid for calibrating viscometer JS2.5 (manufactured by NIPPON GREASE Co., Ltd.). The measurement was carried out three times, and a mean value thereof was then obtained. As a result, the viscosity of Stamp Solution 1-1 was measured to be 1.31 mPa·s.

### (Stamp Solution 1-2)

Stamp Solution 1-2 was obtained in the same manner as that for Stamp Solution 1-1, with the exception that polyvinyl alcohol was not added to the solvent for stamp solution. The viscosity of Stamp Solution 1-2 was obtained in the same manner as that for Stamp Solution 1-1. As a result, the viscosity of Stamp Solution 1-2 was measured to be 1.21 mPa·s.

### (Stamp Solution 1-3)

Stamp Solution 1-3 was obtained in the same manner as that for Stamp Solution 1-1, with the exception that Tween 20 was not added to the solvent for stamp solution. The viscosity of Stamp Solution 1-3 was obtained in the same manner as that for Stamp Solution 1-1. As a result, the viscosity of Stamp Solution 1-3 was measured to be 1.22 mPa·s.

### (Stamp Solution 1-4)

Stamp Solution 1-4 was obtained in the same manner as that for Stamp Solution 1-1, with the exception that polyvinyl alcohol and Tween 20 were not added to the solvent for stamp solution. The viscosity of Stamp Solution 1-4 was obtained in the same manner as that for Stamp Solution 1-1. As a result, the viscosity of Stamp Solution 1-4 was measured to be 1.11 mPa·s.

### (Stamp Solution 1-5)

Morpholineethanesulfonic acid monohydrate was dissolved in ultrapure water to result in 0.53 parts by weight, so as to obtain a MES solution. Polyvinyl alcohol (manufactured by Wako Pure Chemical Industries, Ltd., 160-03055) and Tween 20 (manufactured by Sigma-Aldrich, P7949-100ML) were each dissolved in the HEPES solution to result in 0.1 part by weight and 0.05 parts by weight, respectively, thereby obtaining a solvent for stamp solution. αs1-Casein (manufactured by Sigma-Aldrich, C6780-250MG) used as a material to be immobilized was allowed to react with Linker beads (manufactured by TAMAGAWA SEIKI Co., Ltd.) in the MES buffer at 40°C for 20 hours, and was then purified. The resulting solution was dispersed in the solvent for stamp solution, so as to obtain Stamp Solution 1-5.

### (Stamp Solution 1-6)

Stamp Solution 1-6 was obtained in the same manner as that for Stamp Solution 1-5, with the exception that polyvinyl alcohol was not added to the solvent for stamp solution.

### (Stamp Solution 1-7)

Stamp Solution 1-7 was obtained in the same manner as that for Stamp Solution 1-5, with the exception that Tween 20 was not added to the solvent for stamp solution.

### (Stamp Solution 1-8)

Stamp Solution 1-8 was obtained in the same manner as that for Stamp Solution 1-5, with the exception that polyvinyl alcohol and Tween 20 were not added to the solvent for stamp solution.

### (Stamp Solution 1-9)

Polyvinyl alcohol (manufactured by Wako Pure Chemical Industries, Ltd., 160-03055) and Tween 20 (manufactured by Sigma-Aldrich, P7949-100ML) were each dissolved in the PBS solution to result in 0.1 part by weight and 0.05 parts by weight, respectively thereby obtaining a solvent for stamp solution. αs1-Casein (manufactured by Sigma-Aldrich, C6780-250MG) used as a material to be immobilized was dissolved in the solvent for stamp solution to result in a concentration of 0.1 mg/mL, so as to obtain Stamp Solution 1-9.

### (Stamp Solution 1-10)

Stamp Solution 1-10 was obtained in the same manner as that for Stamp Solution 1-9, with the exception that polyvinyl alcohol was not added to the solvent for stamp solution.

### (Stamp Solution 1-11)

Stamp Solution 1-11 was obtained in the same manner as that for Stamp Solution 1-9, with the exception that Tween 20 was not added to the solvent for stamp solution.

### (Stamp Solution 1-12)

Stamp Solution 1-12 was obtained in the same manner as that for Stamp Solution 1-9, with the exception that polyvinyl alcohol and Tween 20 were not added to the solvent for stamp solution.

Conditions for preparation of each stamp solution were summarized in Table 1.

**Table 1 Conditions for preparation of each stamp solution**

| Stamp Solution | Material to be immobilized | Immobilization carrier | Immobilization reaction solution | Reaction conditions | Solvent for stamp solution |
|---|---|---|---|---|---|
| 1-1 | Biotinylated peptide | Streptavidin beads | PBS buffer | 4°C | HEPES-PVA-Tween |
| | | | | 1.5 hours | |
| 1-2* | Biotinylated peptide | Streptavidin beads | PBS buffer | 4°C | HEPES-Tween |
| | | | | 1.5 hours | |
| 1-3* | Biotinylated peptide | Streptavidin beads | PBS buffer | 4°C | HEPES-PVA |
| | | | | 1.5 hours | |
| 1-4* | Biotinylated peptide | Streptavidin beads | PBS buffer | 4°C | HEPES |
| | | | | 1.5 hours | |
| 1-5 | αs1-casein | Linker beads | 25 mM MES buffer | 40°C | HEPES-PVA-Tween |
| | | | | 20 hours | |
| 1-6* | αs1-casein | Linker beads | 25 mM MES buffer | 40°C | HEPES-Tween |
| | | | | 20 hours | |
| 1-7* | αs1-casein | Linker beads | 25 mM MES buffer | 40°C | HEPES-PVA |
| | | | | 20 hours | |
| 1-8* | αs1-casein | Linker beads | 25 mM MES buffer | 40°C | HEPES |
| | | | | 20 hours | |
| 1-9 | αs1-casein | | | | PBS-PVA-Tween |
| 1-10* | αs1-casein | | | | PBS-Tween |
| 1-11* | αs1-casein | | | | PBS-PVA |
| 1-12* | αs1-casein | | | | PBS |

| | | | | | |
|---|---|---|---|---|---|
| * stamp solution not in accordance with the present invention | | | | | |

### < Example 3: Production of biochip (1) >

Bisazide was dissolved in ultrapure water to prepare a 10 mg/mL bisazide solution. The 10 mg/mL bisazide solution was diluted by 5 times (Bisazide Solution A), or by 5000 times (Bisazide Solution B). Bisazide Solution B was dissolved in each of Stamp Solutions 1-1 to 1-8 to result in 4.8 parts by weight of the Bisazide Solution B. Bisazide Solution A was dissolved in each of Stamp Solutions 1-9 to 1-12 to result in 4.8 parts by weight of the Bisazide Solution A.

Using a stamper (manufactured by Geneqs, Genex Arrayer), the thus obtained 12 types of stamp solutions were each spotted on Biochip Substrate 1 or 2 produced in Example 1, in an amount of 3 spots per stamp solution (6 x 6, 36 spots in total), according to a lattice point-type multipoint dispensing spot method (in the order from Stamp Solution 1-12 to Stamp Solution 1-1). The diameter of a spot was 0.36 to 0.54 µm, and the distance between spots was approximately 1.1 mm. After completion of the spotting, the spots were dried using a vacuum dryer at 0.09 MPa for 10 minutes. After completion of the drying, in order to immobilize the materials to be immobilized using the photocrosslinking agent, the spots were irradiated with ultraviolet rays, using a UV irradiation device (manufactured by UVP, CL-1000) at 120 mW/cm² for 10 minutes, so as to obtain Biochip 1 or 2.

### < Example 4: Measurement using chip >

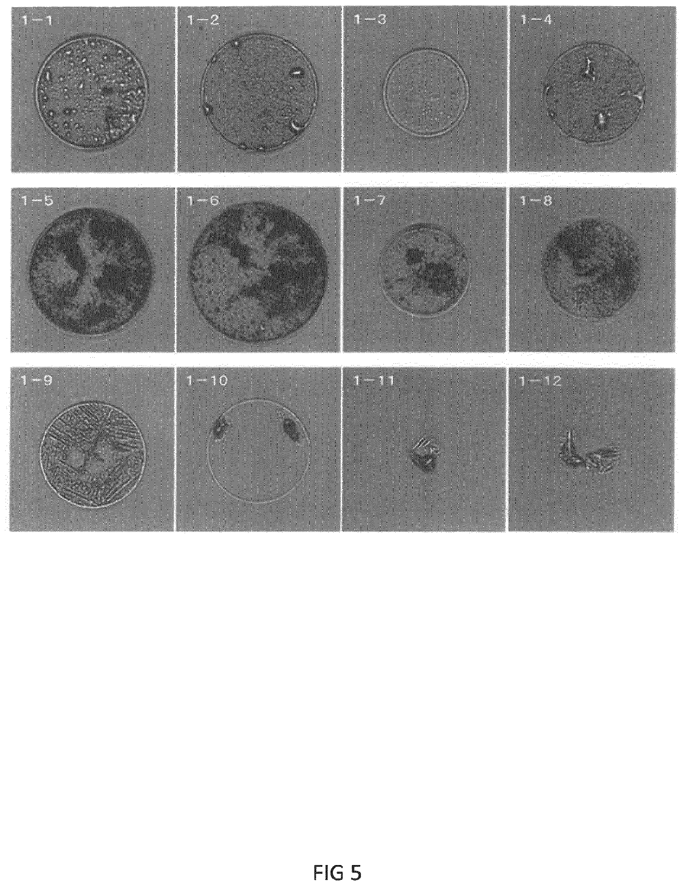

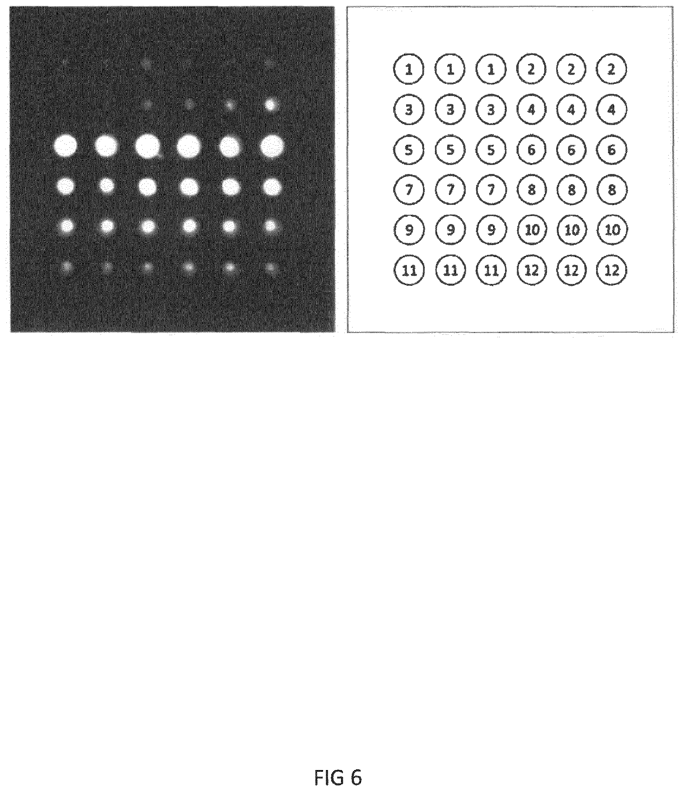

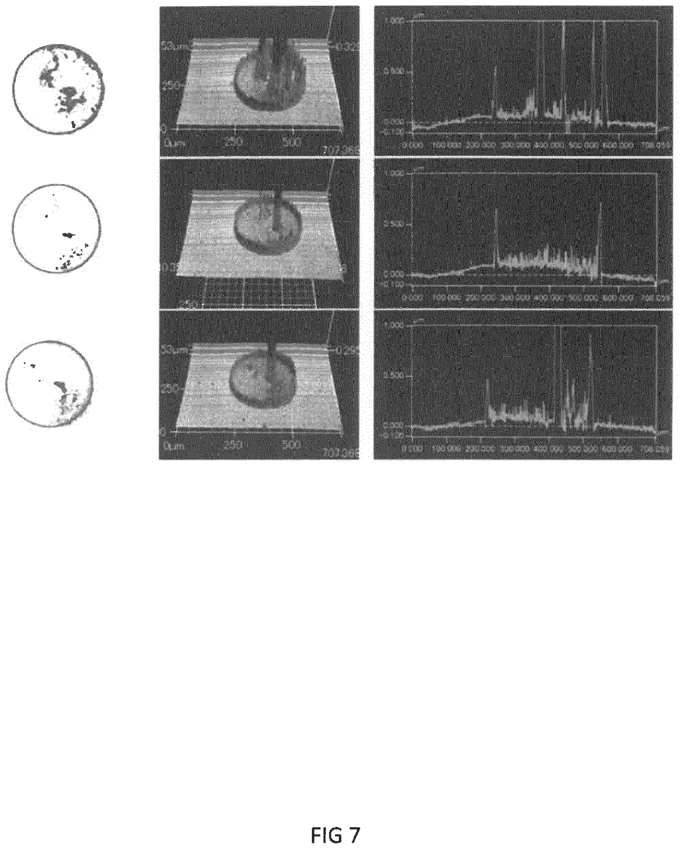

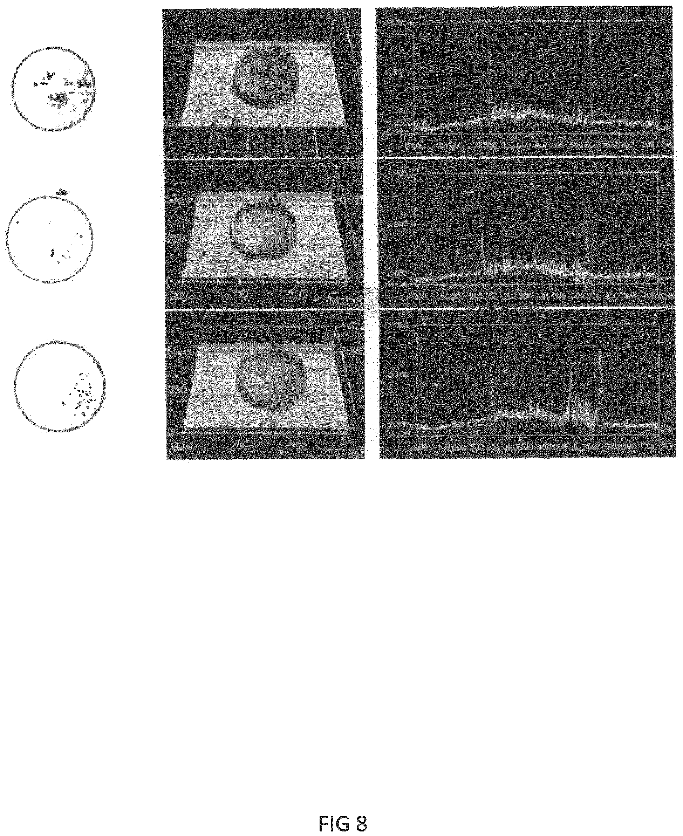

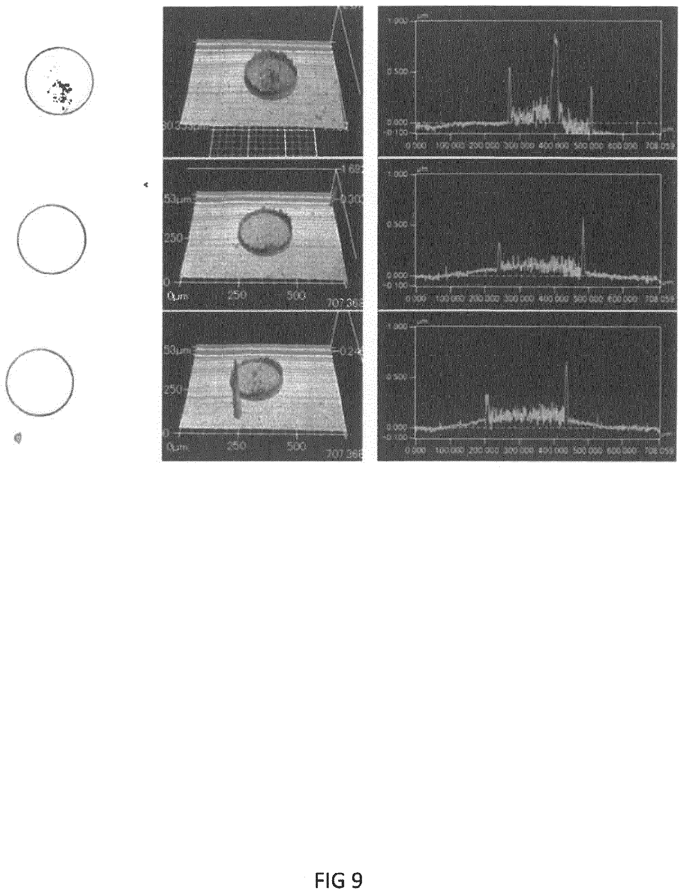

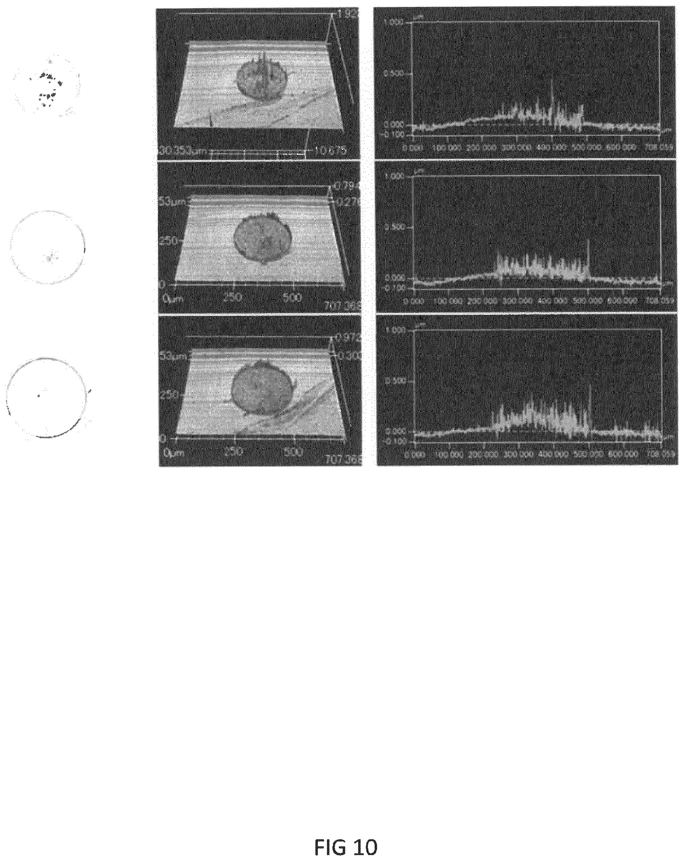

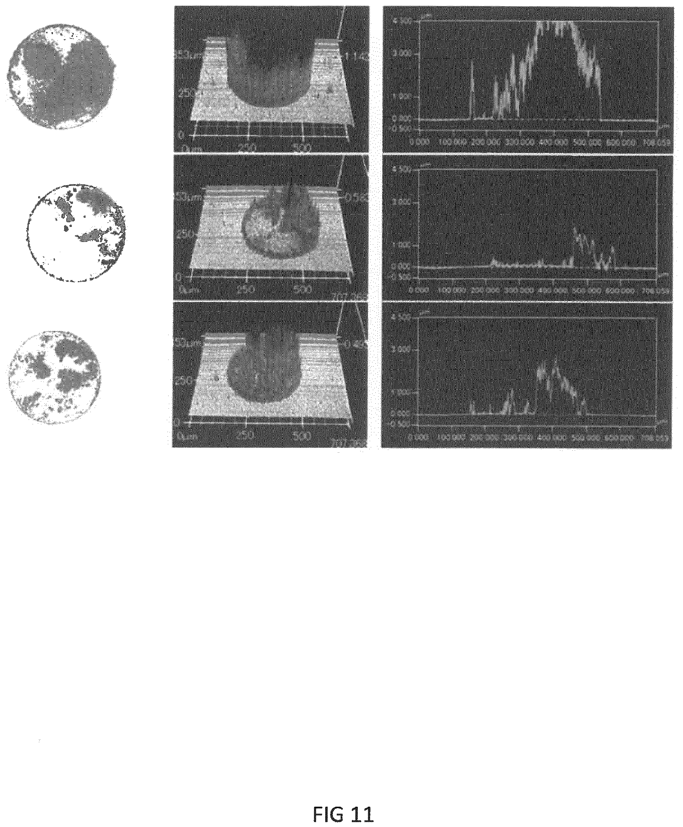

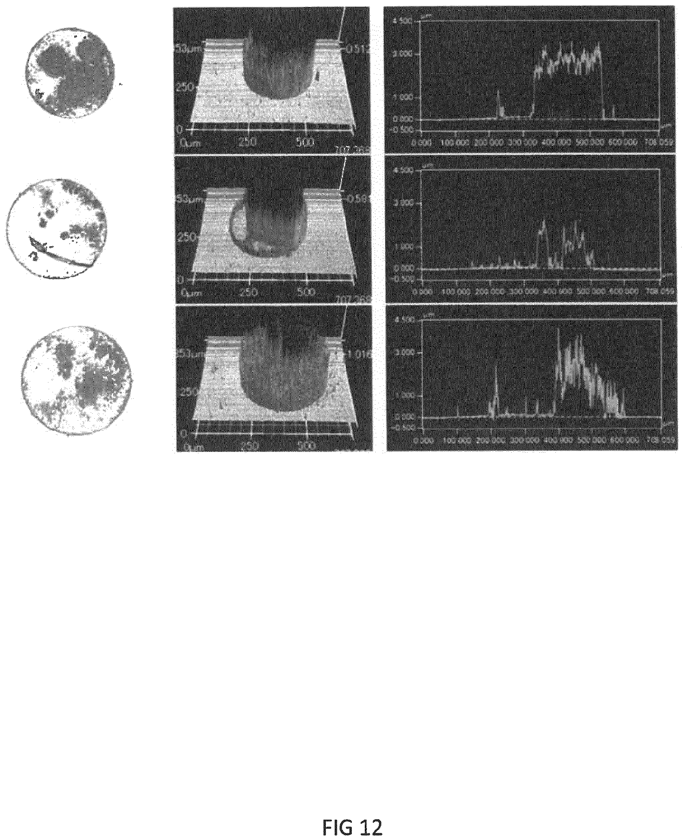

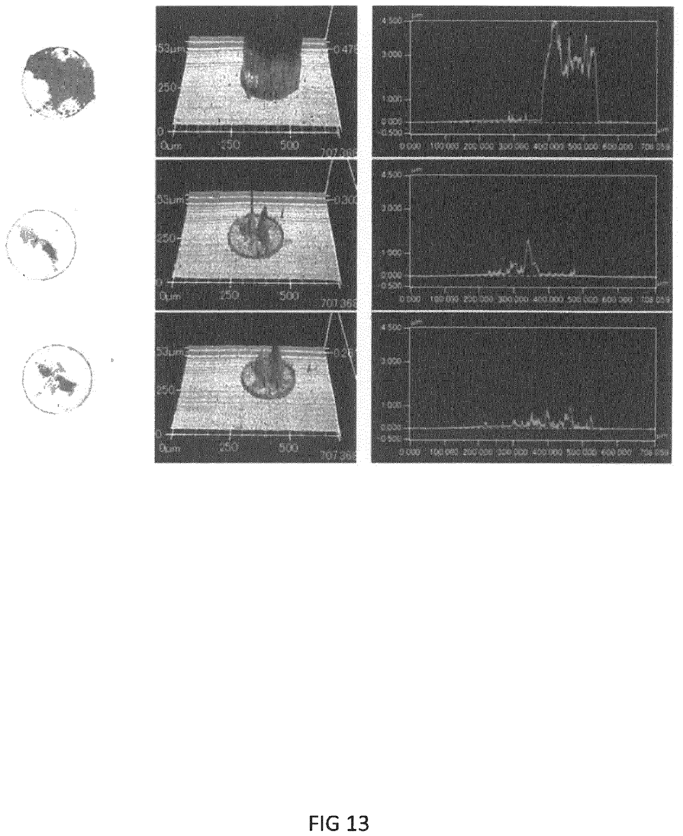

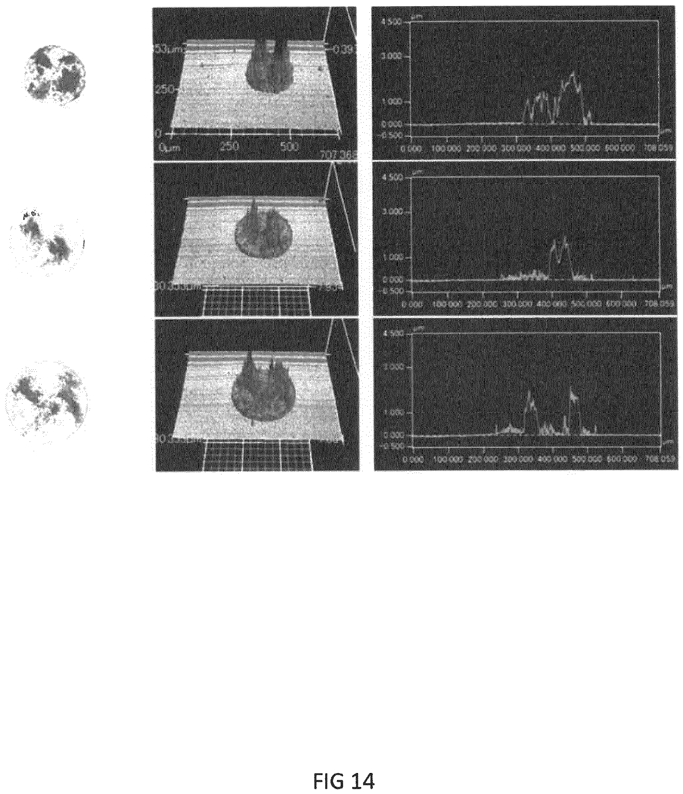

Using the biochip produced in Example 3, an IgG antibody contained in casein-positive human serum (PlasmaLab) was measured. The chip was washed with a TBS-T solution (137 mM sodium chloride, 2.68 mM potassium chloride, 25 mM tris-hydroxymethylaminomethane, pH 7.4, 0.1% by weight of Tween 20) three times. To the reaction area of Biochip 1, 130 µL of 8-fold diluted serum was added as a test sample, and while shaking, it was reacted at room temperature for 8 minutes. The test sample was aspirated and was then washed with a TBS-T solution. After completion of the washing, 130 µL of an anti-human IgG antibody (manufactured by SeraCare Lifesciences, Inc., 0751-1004) that had been 2000 times diluted with Can Get Signal Immunoreaction Enhancer Solution 2 (manufactured by TOYOBO CO., LTD., NKB-301) was added to the resultant, and while shaking, the mixture was reacted at room temperature for 4 minutes. Thereafter, the antibody was aspirated and was then washed with TBS-T. To the resultant, 130 µL of a luminescent reagent (Dynalight Substrate with Rapid Glow Enhancer, Molecular Probes, 4475406) was added, and the thus obtained mixture was then shaken for 1 minute. Thereafter, using SpotSolver manufactured by DYNACOM Co., Ltd. and ImageJ manufactured by National Institutes of Health, the number of pixels in the luminescent portion was counted, while a portion containing no spot was set as a background, so as to measure luminescence intensity. The images obtained with an optical microscope (manufactured by Olympus Corporation, IX71) are shown in Figure 5, and the images of luminescence intensity are shown in Figure 6. The planar images, 3D images, and profile graphs, which were obtained with a laser microscope (manufacture by KEYENCE CORPORATION, VK-X250), are shown in each of Figures 7 to 14. In addition, the luminescence signal intensity (the number of pixels in the luminescent portion) obtained from the image shown in Figure 6 is shown in Table 2, whereas the mean value (n = 3) of the volume of a solid matter (a material to be immobilized + an immobilization carrier) obtained from the 3D images shown in each of Figures 7 to 14 is shown in Table 3.

**Table 2 Luminescence signal intensity measured using biochip**

| Spot | Stamp Solution | Luminescence intensity |
|---|---|---|
| 1 | 1-1 | 8243 |
| 2 | 1-2* | 6242 |
| 3 | 1-3* | 6978 |
| 4 | 1-4* | 7872 |
| 5 | 1-5 | 13 8073 |
| 6 | 1-6* | 128667 |
| 7 | 1-7* | 70302 |
| 8 | 1-8* | 73783 |
| 9 | 1-9 | 29030 |
| 10 | 1-10* | 28050 |
| 11 | 1-11* | 8814 |
| 12 | 1-12* | 11767 |

| | | |
|---|---|---|
| * stamp solution not in accordance with the present invention | | |

**Table 3 Average volume of solid matter immobilized on each spot**

| Spot | Stamp Solution | Average volume (µm³) |
|---|---|---|
| 1 | 1-1 | 40613 |
| 2 | 1-2* | 34995 |
| 3 | 1-3* | 28852 |
| 4 | 1-4* | 24000 |
| 5 | 1-5 | 121800 |
| 6 | 1-6* | 109173 |
| 7 | 1-7* | 63455 |
| 8 | 1-8* | 40006 |

| | | |
|---|---|---|
| * stamp solution not in accordance with the present invention | | |

From the results shown in Figures 5 and 7 to 14, it is found that a spot does not become round when the spot does not contain both a thickener and a surfactant, and that the shape of the spot can be stabilized when the spot contains a thickener and/or a surfactant. From the results shown in Figures 7 to 14 and Table 3, it is found that the volume of the solid matter present on the chip is larger in a spot containing a thickener and/or a surfactant, than in a spot containing neither a thickener nor a surfactant, and thus that larger quantities of immobilization carriers, to which materials to be immobilized bind, are immobilized on the spot containing a thickener and/or a surfactant. Moreover, from the results shown in Figure 6 and Table 2, it is found that the luminescence intensity of a spot containing neither a thickener nor a surfactant is not stabilized and thereby detection sensitivity is decreased, but that the luminescence intensity of a spot can be stabilized when the spot contains a thickener and/or a surfactant, so that detection sensitivity can be improved. Furthermore, the biochip of the present invention enables analysis with an extremely small amount of test sample (16 µL). Further, according to the method for producing a biochip of the present invention, the binding site of a material to be immobilized can be controlled, and a biochip having a stable spot shape can be obtained.

### < Example 5: Production of biochip (2) >

Stamp Solutions 2-1 to 2-8 were prepared in the same manner as that of Example 2, with the exceptions that Streptavidin Mag Sepharose (manufactured by GE Healthcare) was used instead of Streptavidin beads, and Sera-Mag Carboxylate-Modified Magnetic Particles (Hydrophylic) (manufactured by GE Healthcare) were used instead of Linker beads. Conditions for preparation of each stamp solution were summarized in Table 4. Besides, in the table, immobilization carrier "A" indicates Streptavidin Mag Sepharose, and immobilization carrier "B" indicates Sera-Mag Carboxylate-Modified Magnetic Particles.

**Table 4 Conditions for preparation of each stamp solution**

| Stamp Solution | Material to be immobilized | Immobilization carrier | Immobilization reaction solution | Reaction conditions | Solvent for stamp solution |
|---|---|---|---|---|---|
| 2-1 | Biotinylated peptide | A | PBS buffer | 4°C | HEPES-PVA-Tween |
| | | | | 1.5 hours | |
| 2-2* | Biotinylated peptide | A | PBS buffer | 4°C | HEPES-Tween |
| | | | | 1.5 hours | |
| 2-3* | Biotinylated peptide | A | PBS buffer | 4°C | HEPES-PVA |
| | | | | 1.5 hours | |
| 2-4* | Biotinylated peptide | A | PBS buffer | 4°C | HEPES |
| | | | | 1.5 hours | |
| 2-5 | αs1-casein | B | 25 mM MES buffer | 40°C | HEPES-PVA-Tween |
| | | | | 20 hours | |
| 2-6* | αs1-casein | B | 25 mM MES buffer | 40°C | HEPES-Tween |
| | | | | 20 hours | |
| 2-7* | αs1-casein | B | 25 mM MES buffer | 40°C | HEPES-PVA |
| | | | | 20 hours | |
| 2-8* | αs1-casein | B | 25 mM MES buffer | 40°C | HEPES |
| | | | | 20 hours | |

| | | | | | |
|---|---|---|---|---|---|
| * stamp solution not in accordance with the present invention | | | | | |

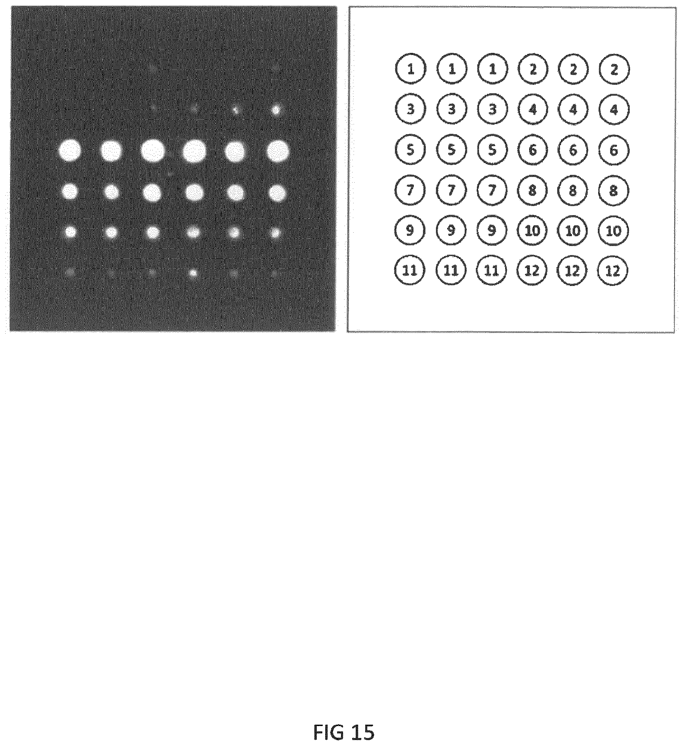

Stamp Solutions 2-1 to 2-8 and 1-9 to 1-12 were each spotted on Biochip Substrate 1 or 2 produced in Example 1 in the same manner as that of Example 3, and thereafter, drying and UV irradiation were also carried out in the same manner as that of Example 3, so as to obtain Biochip 3 or 4. Using these biochips, the same measurement as in Example 4 was carried out. The image of luminescence intensity is shown in Figure 15, and luminescence signal intensity obtained from the image of Figure 15 is shown in Table 5. Observation was also carried out using an optical microscope and a laser microscope, and the same results as those of Example 4 were obtained. In Table 5, the numerical value of Spot 4 is higher than the numerical values of Spots 1 and 2, and thus, the results are different from the results of Spot 12 and Spots 9 and 10, and the results of Spot 8 and Spots 5 and 6, wherein these spots have the same relationship as the relationship of Spot 4 and Spots 1 and 2. This is considered because, in the process of spotting the stamp solutions in the order from Stamp Solution 1-12 to Stamp Solution 1-9, and then, from Stamp Solution 2-8 to Stamp Solution 2-1, Stamp Solution 2-5 that had slightly remained in the stamper might have been mixed into Stamp Solution 2-4 to be spotted next, and the thus obtained stamp solution might have been directly spotted on Spot 4.

**Table 5 Luminescence signal intensity measured using biochip**

| Spot | Stamp Solution | luminescence intensity |
|---|---|---|
| 1 | 2-1 | 4904 |
| 2 | 2-2* | 4230 |
| 3 | 2-3* | 3239 |
| 4 | 2-4* | 15317 |
| 5 | 2-5 | 125994 |
| 6 | 2-6* | 118088 |
| 7 | 2-7* | 67978 |
| 8 | 2-8* | 69867 |
| 9 | 1-9 | 30132 |
| 10 | 1-10* | 28659 |
| 11 | 1-11* | 10116 |
| 12 | 1-12* | 12529 |

| | | |
|---|---|---|
| * stamp solution not in accordance with the present invention | | |

### < Example 6: Preservation of biochip (1) >

### (1) Preparation of buffers

Sodium chloride, potassium chloride, disodium hydrogen phosphate dodecahydrate, and potassium dihydrogen phosphate were each dissolved in ultrapure water to result in 0.8 parts by weight, 0.02 parts by weight, 0.29 parts by weight, and 0.02 parts by weight, respectively, so as to obtain a PBS solution.

4-(2-Hydroxyethyl)-1-piperazineethanesulfonic acid was dissolved in ultrapure water to result in 0.59 parts by weight, so as to obtain a HEPES solution.

Morpholineethanesulfonic acid monohydrate was dissolved in ultrapure water to result in 0.53 parts by weight, so as to obtain a MES solution.

### (2) Preparation of immobilization reaction solution

t-Butyl alcohol (manufactured by Wako Pure Chemical Industries, Ltd., 028-03386) was mixed with dimethyl sulfoxide at a volume ratio of 4 : 1, and thereafter, tris[(1-benzyl-1H-1,2,3-triazol-4-yl)methyl]amine (manufactured by Sigma-Aldrich, 678937-50MG) was dissolved in the mixed solution to result in 0.27 parts by weight, so as to obtain a TBTA solution.

### (3) Preparation of solvents for stamping

Polyvinyl alcohol (manufactured by Wako Pure Chemical Industries, Ltd., 160-03055) and Tween 20 (manufactured by Sigma-Aldrich, P7949-100ML) were each dissolved in a PBS solution to result in 0.1 part by weight and 0.05 parts by weight, respectively, so as to obtain Solution A.

Polyvinyl alcohol (manufactured by Wako Pure Chemical Industries, Ltd., 160-03055) and Tween 20 (manufactured by Sigma-Aldrich, P7949-100ML) were each dissolved in a 25 mM HEPES solution to result in 0.1 part by weight and 0.05 parts by weight, respectively, so as to obtain Solution B.

As materials to be immobilized, αs1-casein (manufactured by Sigma-Aldrich, C6780-250MG), β-casein (manufactured by Sigma-Aldrich, C6905-250MG), both of which were used in a concentration of 0.1 mg/mL, α-lactalbumin (manufactured by Sigma-Aldrich, L5385-25MG), β-lactoglobulin (manufactured by Sigma-Aldrich, L3908-250MG), ovomucoid (manufactured by Sigma-Aldrich, T2011-250MG), and ovalbumin (manufactured by Sigma-Aldrich, A2512-250MG) were used. The materials to be immobilized were each dissolved in Solution A, so as to obtain Stamp Solutions 3-1 to 3-6. As materials to be immobilized, αs1-casein, α-lactalbumin, and ovomucoid were each allowed to react with Linker beads in a MES buffer at 40°C for 20 hours, and were then purified, followed by dispersion in Solution B, so as to obtain Stamp Solutions 3-7 to 3-9. On the other hand, only Linker beads, on which the materials to be immobilized were not immobilized, were dispersed in Solution B to obtain Stamp Solution 3-10. As materials to be immobilized, Biotinylated Peptide A and Biotinylated Peptide B, which had been formed by biotinylating the termini of different Partial Peptides A and B of αs1-casein (each consisting of 15 amino acid residues), were each allowed to react with Streptavidin beads in a PBS solution at 4°C for 1.5 hours, and were then purified, followed by dispersion in Solution B, so as to obtain Stamp Solutions 3-11 and 3-12. Likewise, Azidated Peptide A and Azidated Peptide B, which had been formed by azidating the termini of the Peptides A and B, were each allowed to react with Alkyne beads (manufactured by TAMAGAWA SEIKI Co., Ltd.) in a HEPES solution at 25°C for 20 hours, and were then purified, followed by dispersion in Solution B, so as to obtain Stamp Solutions 3-13 and 3-14. Conditions for preparation of each stamp solution were summarized in Table 6.

**Table 6 Conditions for preparation of each stamp solution**

| Stamp Solution | Material to be immobilized | Immobilization carrier | Immobilization reaction solution | Reaction conditions | Solvent for stamping |
|---|---|---|---|---|---|
| 3-1 | αs1-casein | | | | Solution A |
| 3-2 | β-casein | | | | Solution A |
| 3-3 | α-lactalbumin | | | | Solution A |
| 3-4 | β-lactoglobulin | | | | Solution A |
| 3-5 | Ovomucoid | | | | Solution A |
| 3-6 | Ovalbumin | | | | Solution A |
| 3-7 | αs1-casein | Linker beads | 25 mM MES buffer | 40°C | Solution B |
| | | | | 20 hours | |
| 3-8 | α-lactalbumin | Linker beads | 25 mM MES buffer | 40°C | Solution B |
| | | | | 20 hours | |
| 3-9 | Ovomucoid | Linker beads | 25 mM MES buffer | 40°C | Solution B |
| | | | | 20 hours | |
| 3-10 | | Linker beads | 25 mM MES buffer | 40°C | Solution B |
| | | | | 20 hours | |
| 3-11 | Biotinylated peptide A | Streptavidin beads | PBS buffer | 4°C | Solution B |
| | | | | 1.5 hours | |
| 3-12 | Biotinylated peptide B | Streptavidin beads | PBS buffer | 4°C | Solution B |
| | | | | 1.5 hours | |
| 3-13 | Azidated peptide A | Alkyne beads | TBTA solution | 25°C | Solution B |
| | | | | 20 hours | |
| 3-14 | Azidated peptide B | Alkyne beads | TBTA solution | 25°C | Solution B |
| | | | | 20 hours | |

### (4) Production of biochip

Bisazide was dissolved in ultrapure water to prepare a 10 mg/mL bisazide solution. The 10 mg/mL bisazide solution was diluted by 5 times (Bisazide Solution A), or by 5000 times (Bisazide Solution B). Bisazide Solution A was dissolved in each of Stamp Solutions 3-1 to 3-6 to result in 4.8 parts by weight of the Bisazide Solution A. With regard to Stamp Solutions 3-7 to 3-14, beads were dispersed in each of the stamp solutions, and Bisazide Solution B was then dissolved therein to result in 4.8 parts by weight of the Bisazide Solution B.

Using a stamper (manufactured by Geneqs, Genex Arrayer), the thus obtained 14 types of stamp solutions were each spotted on Biochip Substrate 1 produced in Example 1, in an amount of 3 spots per stamp solution (6 x 7, 42 spots in total), according to a lattice point-type multipoint dispensing spot method. After completion of the spotting, the spots were dried using a vacuum dryer at 0.09 MPa for 10 minutes. After completion of the drying, in order to immobilize the materials to be immobilized using the photocrosslinking agent, the spots were irradiated with ultraviolet rays, using a UV irradiation device (manufactured by UVP, CL-1000) at 120 mW/cm² for 10 minutes, so as to obtain Biochip 3.

### (5) Preservation of biochip

The produced Biochip 3 was preserved under the following conditions.
- Preservation Example 1: The biochip, together with an oxygen scavenger (AGELESS manufactured by MITSUBISHI GAS CHEMICAL COMPANY, INC.), was enclosed in an aluminum bag manufactured by MITSUBISHI GAS CHEMICAL COMPANY, INC. The bag was placed in an incubator at 25°C, and was then preserved for 2 weeks.
- Preservation Example 2: The biochip was preserved in the same manner as that of Preservation Example 1, with the exception that the oxygen scavenger was not enclosed in the bag, after the biochip had been enclosed therein, and then, the bag was evacuated and sealed.
- Preservation Example 3: The biochip was preserved in the same manner as that of Preservation Example 1, with the exception that the oxygen scavenger was not enclosed in the bag.

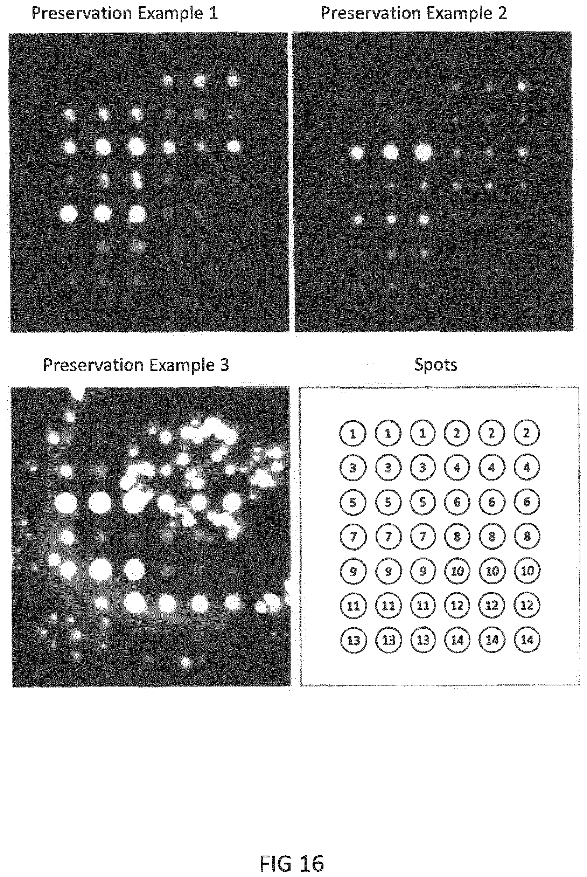

Using the biochips obtained in Preservation Examples 1 to 3, the same measurement as in Example 4 was carried out. The measurement results of luminescence intensity are shown in Figure 16, and luminescence signal intensity obtained from the image of Figure 16 is shown in Table 7.

**Table 7 Luminescence signal intensity measured using biochip preserved for 2 weeks**

| Spot | Stamp Solution | Preserved for 2 weeks | | |
|---|---|---|---|---|
| | | Room temperature | | |
| | | Preservation Example 1 | Preservation Example 2 | Preservation Example 3 |
| 1 | 3-1 | 1226.1 | 825.7 | Unmeasurable |
| 2 | 3-2 | 26710.0 | 17276.0 | |
| 3 | 3-3 | 14346.7 | 8093.8 | |
| 4 | 3-4 | 11833.5 | 10515.1 | |
| 5 | 3-5 | 61081.9 | 48322.1 | |
| 6 | 3-6 | 25365.7 | 23272.4 | |
| 7 | 3-7 | 13341.8 | 9803.9 | |
| 8 | 3-8 | 13929.0 | 19054.1 | |
| 9 | 3-9 | 60350.0 | 33347.2 | |
| 10 | 3-10 | 7350.4 | 5667.5 | |
| 11 | 3-11 | 15098.4 | 17731.9 | |
| 12 | 3-12 | 5942.5 | 6411.4 | |
| 13 | 3-13 | 13316.9 | 12772.0 | |
| 14 | 3-14 | 5178.9 | 5894.4 | |

From the results of Preservation Examples 1 and 2 shown in Figure 16 and Table 7, it is found that the stability of the biochip of the present invention is maintained, even after long-term preservation, by being preserved in a deoxygenated state or in vacuum. On the other hand, from the results of Preservation Example 3 shown in Figure 16 and Table 7, it is found that, in the case of a biochip to which the preservation method had not been applied, the surface of the chip was deteriorated, and luminescence intensity could not be read.

### < Example 7: Production of biochip substrate (2) >

### < Substrate 1 >

A polycarbonate sheet (manufactured by MITSUBISHI GAS CHEMICAL COMPANY, INC., MR58U, thickness: 0.8 mm) was irradiated with ultraviolet rays, using a UV-ozone irradiation device (manufactured by SEN LIGHTS Co., Ltd., SSP16-110, UV lamp: SUV110GS-36L) at an irradiation distance of 50 mm for 2 minutes, so that the entire upper surface of the sheet was hydrophilized. Using an adhesive, a silicon rubber-made O ring (14 ϕ) was adhered to the hydrophilized sheet to form a reaction area, so as to obtain Biochip Substrate 1.

### < Substrate 2 >

Biochip Substrate 2 was obtained in the same manner as that for Substrate 1, with the exception that the treatment using a UV-ozone irradiation device was carried out for 1 minute.

### < Substrate 3 >

Biochip Substrate 3 was obtained in the same manner as that for Substrate 1, with the exception that the treatment using a UV-ozone irradiation device was carried out for 30 seconds.

### < Substrate 4 >

Biochip Substrate 4 was obtained in the same manner as that for Substrate 1, with the exception that the treatment using a UV-ozone irradiation device was carried out for 8 minutes.

### < Substrate 5 >

Biochip Substrate 5 was obtained in the same manner as that for Substrate 1, with the exceptions that a desktop semi-automatic atmospheric pressure plasma surface reforming device (manufactured by Well, MyPL-Auto100) was used instead of the UV-ozone irradiation device, and that the treatment was carried out once at 100 W and at 50 mm/sec.

### < Substrate 6 >

Biochip Substrate 6 was obtained in the same manner as that of Example 1, with the exceptions that the UV-ozone treatment was not carried out.

### < Substrate 7 >

Polyethylene glycol monomethacrylate (manufactured by Sanyu Chemical Co., Ltd.; the molecular weight of polyethylene glycol moiety: 350) and 4,4'-diazidostilbene-2,2'-disulfonic acid (manufactured by TOKYO CHEMICAL INDUSTRY CO., LTD.; 98%; hereinafter referred to as "bisazide") were each dissolved in a 75% ethanol aqueous solution to result in 0.5 parts by weight and 0.025 parts by weight, respectively, so as to prepare a coating solution. Instead of the treatment using a UV-ozone irradiation device, a polycarbonate sheet was coated with 15 µL of the above-prepared coating solution, using a spin-coater (manufactured by MIKASA, MS-A100), under conditions of 800 rpm for 5 seconds and 5000 rpm for 10 seconds. Thereafter, using a UV irradiation device (manufactured by UVP, CL-1000), the sheet was irradiated with ultraviolet rays at 120 mW/cm² for 10 minutes, so that bisazide was crosslinked to form a coating layer of a non-specific adsorption preventing agent. With these exceptions, Biochip Substrate 7 was obtained in the same manner as that for Substrate 1.

Functional groups on the surface of the resin were measured using an infrared spectrophotometer (manufactured by JASCO Corporation, FT/IR-4200). The state of the resin surface is shown by using the ratio of the absorption intensity (D_{OH}) of the characteristic absorption (3200 to 3600 cm⁻¹) of -OH contained in hydroxyl groups and carboxyl groups, or the absorption intensity (D_{CO}) of characteristic absorption (1600 to 1950 cm⁻¹) of C=O contained in carboxyl groups and carbonyl groups, to the absorption intensity (D_{CH}) of the characteristic absorption (2900 to 3100 cm⁻¹) of CH as a main structure of the substrate.

D_{OH}/D_{CH} and D_{CO}/D_{CH} of Biochip Substrates 1 to 6 are shown in Table 8.

**Table 8 Functional groups on surface of biochip substrate**

| | D_{OH}/D_{CH} | D_{CO}/D_{CH} |
|---|---|---|
| Substrate 1 | 0.68 | 0.93 |
| Substrate 2 | 0.60 | 0.88 |
| Substrate 3 | 0.52 | 0.81 |
| Substrate 4 | 0.77 | 1.01 |
| Substrate 5 | 0.49 | 0.83 |
| Substrate 6* | 0.45 | 0.76 |

| | | |
|---|---|---|
| * substrate not in accordance with the present invention | | |

### < Measurement of contact angle >

The contact angle was measured using a contact angle measurement device (manufactured by Kyowa Interface Science, Inc., Drop Master 500), and was then calculated according to a θ/2 method (wherein the contact angle was calculated at θ/2 = arctan (h/r), wherein θ indicates a contact angle, r indicates the radius of a droplet, and h indicates the height of a droplet). The contact angle is indicated as a mean value of the contact angles measured at three sites. Herein, as a first measurement point, the central portion of the sample was selected, and as second and third measurement points, two points, which were 20 mm or more apart from the first measurement point and were at positions point-symmetric to the first measurement point, were selected.

The contact angles of Biochip Substrates 1 to 7 are shown in Table 9.

**Table 9 Contact angle of biochip substrate**

| | contact angle/° |
|---|---|
| Substrate 1 | 25 |
| Substrate 2 | 43 |
| Substrate 3 | 66 |
| Substrate 4 | 24 |
| Substrate 5 | 63 |
| Substrate 6* | 81 |
| Substrate 7 | 25 |

| | |
|---|---|
| * substrate not in accordance with the present invention | |

As shown in Table 8 and Table 9, it is found that hydroxyl groups and/or carboxyl groups and/or carbonyl groups present on the surface are increased in Biochip Substrates 1 to 5, in comparison to Biochip Substrate 6, and that the contact angle is increased.

### < Example 8: Production of biochip (3) >

Using a stamper (manufactured by Geneqs, Genex Arrayer), 14 types of stamp solutions, namely, Stamp Solutions 3-1 to 3-14 prepared in Example 6 were each spotted on Biochip Substrates 1 to 7, in an amount of 3 spots per stamp solution (6 x 7, 42 spots in total), according to a lattice point-type multipoint dispensing spot method. After completion of the spotting, the spots were dried using a vacuum dryer at 0.09 MPa for 10 minutes. After completion of the drying, in order to immobilize the materials to be immobilized using the photocrosslinking agent, the spots were irradiated with ultraviolet rays, using a UV irradiation device (manufactured by UVP, CL-1000) at 120 mW/cm² for 10 minutes to obtain a biochip.

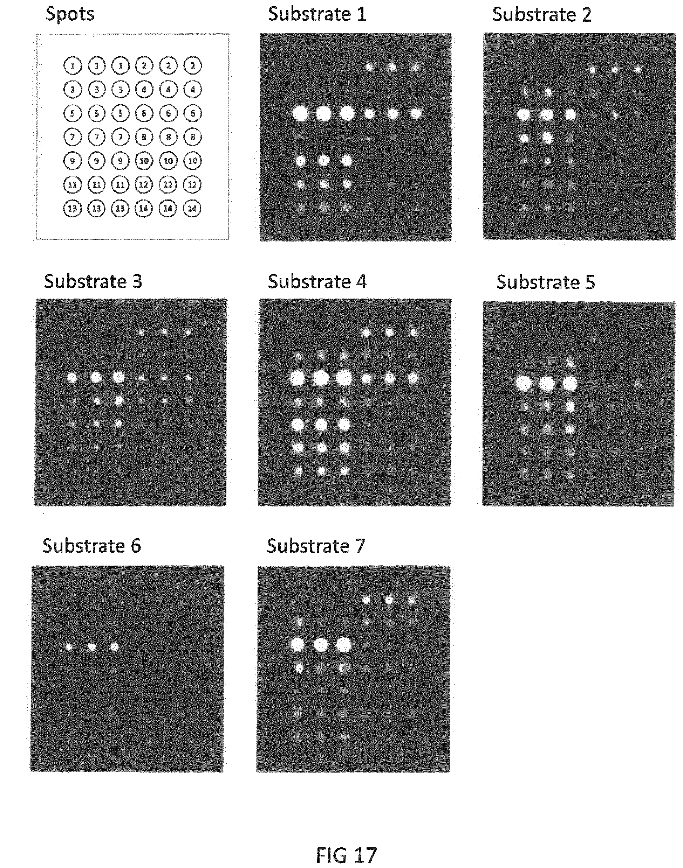

Using the obtained biochip, luminescence intensity was measured in the manner as that of Example 4. The measurement results of the luminescence intensity of each of the biochips produced with Biochip Substrates 1 to 7 are shown in Figure 17, and luminescence signal intensity obtained from the images of Figure 17 is shown in Table 10. The luminescence signal intensity indicates the number of pixels in the luminescent portion.

**Table 10 Luminescence signal intensity measured using each biochip**

| Spot | Stamp Solution | Substrate 1 | Substrate 2 | Substrate 3 | Substrate 4 | Substrate 5 | Substrate 6* | Substrate 7 |
|---|---|---|---|---|---|---|---|---|
| 1 | 3-10 | 2188 | 1692 | 1098 | 4230 | 2041 | 535 | 2688 |
| 2 | 3-6 | 27018 | 23965 | 24556 | 33205 | 6192 | 7584 | 30306 |
| 3 | 3-9 | 5336 | 16678 | 9762 | 24946 | 15481 | 4505 | 14646 |
| 4 | 3-5 | 7463 | 10047 | 10179 | 19191 | 1761 | 2729 | 17560 |
| 5 | 3-7 | 123688 | 57847 | 56568 | 183368 | 94514 | 26997 | 75058 |
| 6 | 3-1 | 64576 | 14716 | 25807 | 50154 | 14051 | 3071 | 10196 |
| 7 | 3-8 | 8158 | 34171 | 31202 | 31091 | 27790 | 7251 | 27180 |
| 8 | 3-3 | 8719 | 5513 | 21925 | 15071 | 9870 | 1622 | 18019 |
| 9 | 3-2 | 66746 | 21859 | 31588 | 77260 | 25619 | 2453 | 16530 |
| 10 | 3-4 | 4792 | 1702 | 8037 | 10749 | 2683 | 402 | 4599 |
| 11 | 3-11 | 27903 | 16513 | 22308 | 38240 | 17180 | 5660 | 19134 |
| 12 | 3-12 | 8807 | 6917 | 8381 | 15774 | 6688 | 4416 | 9047 |
| 13 | 3-13 | 23528 | 17847 | 15393 | 31443 | 20848 | 5413 | 20723 |
| 14 | 3-14 | 11245 | 5571 | 7225 | 15855 | 7270 | 2376 | 7983 |

| | | | | | | | | |
|---|---|---|---|---|---|---|---|---|
| * substrate not in accordance with the present invention | | | | | | | | |

As shown in Figure 17 and Table 10, sufficient luminescence intensity cannot be obtained from the biochip produced using Biochip Substrate 6. On the other hand, in the case of Biochip Substrates 1 to 5 each having a resin-made surface comprising a hydrophilic reaction area, materials to be immobilized can be immobilized thereon without using a coating layer, and when compared with Biochip Substrate 7 coated with a water-soluble polymer, Biochip Substrates 1 to 5 exhibit luminescence intensity equivalent to or greater than that of Biochip Substrate 7. Accordingly, by using a biochip substrate having a resin-made surface comprising a hydrophilic reaction area, there can be provided a highly sensitive biochip, in which materials to be immobilized can be immobilized using a photocrosslinking agent in an amount equal to the case of coating the biochip with a non-specific adsorption preventing layer, without using such a non-specific adsorption preventing layer such as a water-soluble polymer, and which is excellent in terms of luminescence intensity and suppresses the non-specific adsorption of a material to be detected.

### < Example 9: Preservation of biochip (2) >

### Preservation Example 4

A biochip, which had been produced in the same manner as that of Example 8 using Biochip Substrate 1 and the stamp solution shown in Table 6, was enclosed, together with an oxygen scavenger (AGELESS, manufactured by MITSUBISHI GAS CHEMICAL COMPANY, INC.), in an incubator at 25°C, and was then preserved for 2 weeks.

### Preservation Example 5

A biochip produced in the same manner as that of Preservation Example 4 was preserved in the same manner as that of Preservation Example 4, with the exception that the preservation period was set at 8 weeks.

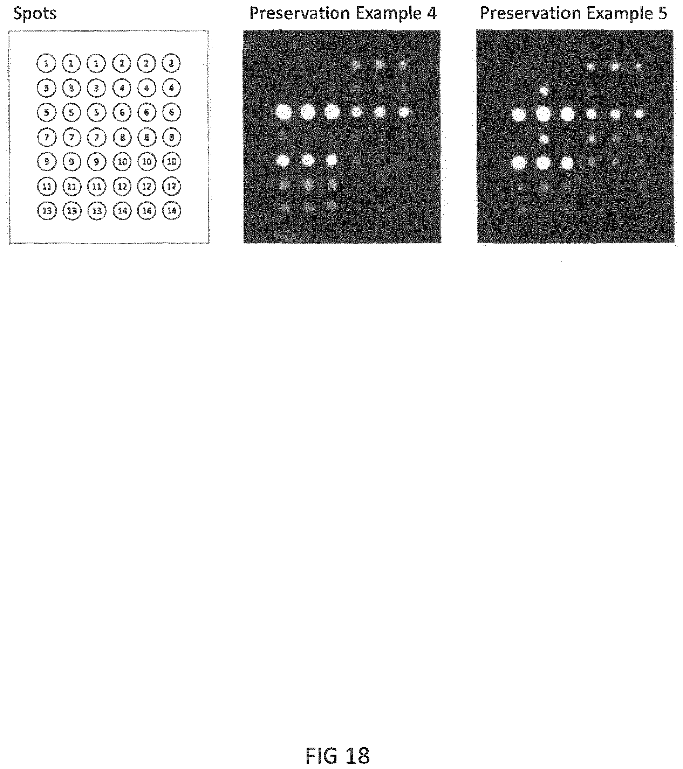

The luminescence intensity of the biochips obtained in Preservation Examples 4 and 5 was measured in the same manner as that of Example 4. The measurement results of the luminescence intensity are shown in Figure 18, and luminescence signal intensity obtained from the images of Figure 18 is shown in Table 11.

**Table 11 Luminescence signal intensity measured using biochip**

| Spot | Stamp Solution | Preservation Example 4 | Preservation Example 5 |
|---|---|---|---|
| 1 | 3-10 | 1424 | 2047 |
| 2 | 3-6 | 23693 | 26589 |
| 3 | 3-9 | 5319 | 5676 |
| 4 | 3-5 | 10240 | 9496 |
| 5 | 3-7 | 58736 | 83757 |
| 6 | 3-1 | 51994 | 54491 |
| 7 | 3-8 | 6325 | 3455 |
| 8 | 3-3 | 11207 | 19528 |
| 9 | 3-2 | 66110 | 94606 |
| 10 | 3-4 | 6709 | 17529 |
| 11 | 3-11 | 20768 | 16129 |
| 12 | 3-12 | 6398 | 6755 |
| 13 | 3-13 | 17452 | 13085 |
| 14 | 3-14 | 7395 | 6637 |

From Figure 18 and Table 11, it is found that the stability of the biochip of the present invention is maintained, even after long-term preservation, by being preserved in a deoxygenated state.

By using the biochip of the present invention, on which different types of biomolecules are immobilized in the form of a plurality of spots, multiple items can be simultaneously analyzed with a small amount of test sample, at high speed, at low costs and with easy handling ability, and thus, the biochip of the present invention can be utilized in point of care testing analysis that is to be applied in clinical sites, etc.

Moreover, according to the present invention, a material to be immobilized can be immobilized on the substrate of the biochip without the need of being coated with a coating layer such as a water-soluble polymer, and the substrate is excellent in terms of luminescence intensity and also, can suppress the non-specific adsorption of a material to be detected. Thereby, it becomes possible to inexpensively produce the biochip of the present invention by a simple production process.

### Reference Signs List

10: Biochip (A)
11: Biochip (B)
12: Biochip (C)
13: Biochip (D)
101: Substrate
101A: Upper surface of substrate
102: Boundary
103: Reaction area
104: Coating layer
105: Spot

## Claims

1. A biochip having a substrate with a hydrophilic reaction area and a spot of a material to be immobilized comprising a biological material that are disposed in the reaction area,
wherein the spot further comprises a thickener and a surfactant,
wherein the contact angle of the hydrophilic reaction area measured by a static contact angle measurement method is 1° or more and less than 80°,
wherein the thickener is gellan gum, xanthan gum, curdlan, pullulan, a guar gum derivative, locust bean gum, carrageenan, pectin, tamarind gum, psyllium seed gum, dextran, glycerin, carboxymethyl cellulose, hydroxyethyl cellulose, hydroxymethylpropyl cellulose, lanolin, methyl cellulose, Vaseline, polyethylene glycol, polyvinyl alcohol, polyvinyl pyrrolidone, a carboxyl vinyl polymer, polyvinyl pyrrolidone, polyvinyl alcohol, dextrin acid fatty ester, inulin acid fatty ester, or a mixture of two or more types selected from these,
wherein the surfactant is a nonionic surfactant, and
wherein the spot is formed by
a step of allowing a liquid comprising the material to be immobilized, a photocrosslinking agent having at least two photoreactive groups in a single molecule, and the thickener and the surfactant, to adhere, in the form of grid-like spots, to the substrate; and
a step of immobilizing the material to be immobilized adhering to the substrate thereon.

2. The biochip according to claim 1, wherein the reaction area is surrounded by a boundary capable of retaining a liquid therein.

3. The biochip according to claim 1 or 2, wherein the substrate has a resin-made surface comprising the hydrophilic reaction area.

4. The biochip according to claim 3, wherein the reaction area is covered with functional groups having polarity selected from a hydroxyl group, a carbonyl group, a carboxyl group, a methoxy group, a peroxide group, and a polar ether group, that are generated as a result that bonds associated with carbon of the resin are cleaved and the cleaved sites bind with oxygen.

5. The biochip according to claim 1 or 2, wherein the material to be immobilized is immobilized on the substrate via a coating layer retaining the material to be immobilized.

6. The biochip according to claim 5, wherein the coating layer consists of a water-soluble polymer.

7. The biochip according to claim 6, wherein the water-soluble polymer is polyethylene glycol methacrylate.

8. The biochip according to any one of claims 1 to 7, wherein the material to be immobilized is a peptide, a nucleic acid, a sugar chain, or a mixture of one or more types selected from these.

9. The biochip according to any one of claims 1 to 8, wherein the material to be immobilized is immobilized on a microparticle carrier as an immobilization carrier, and the immobilization carrier on which the material to be immobilized is immobilized is immobilized on the reaction area.

10. The biochip according to any one of claims 1 to 9, wherein the surfactant is Triton X-100, Tween 20, Tween 80, or a mixture of two or more types selected from these.

11. The biochip according to any one of claims 1 to 10, wherein the substrate is any resin material of polystyrene, polypropylene, polycarbonate, and acrylic resins.

12. A method for producing the biochip according to claim 1, comprising:
a step of forming a hydrophilic reaction area; and
a step of forming a spot of the material to be immobilized comprising a biological material on the reaction area,
wherein the spot comprises a thickener and a surfactant,
wherein the contact angle of the hydrophilic reaction area measured by a static contact angle measurement method is 1° or more and less than 80°,
wherein the thickener is gellan gum, xanthan gum, curdlan, pullulan, a guar gum derivative, locust bean gum, carrageenan, pectin, tamarind gum, psyllium seed gum, dextran, glycerin, carboxymethyl cellulose, hydroxyethyl cellulose, hy-droxymethylpropyl cellulose, lanolin, methyl cellulose, Vaseline, polyethylene glycol, polyvinyl alcohol, polyvinyl pyrrolidone, a carboxyl vinyl polymer, polyvinyl pyrrolidone, polyvinyl alcohol, dextrin acid fatty ester, inulin acid fatty ester, or a mixture of two or more types selected from these,
wherein the surfactant is a nonionic surfactant, and
wherein the step of forming a spot comprises:
a step of allowing a liquid comprising the material to be immobilized, a photocrosslinking agent having at least two photoreactive groups in a single molecule, and the thickener and the surfactant, to adhere, in the form of grid-like spots, to the substrate; and
a step of immobilizing the material to be immobilized adhering to the substrate thereon.

13. A package prepared by packaging the biochip according to any one of claims 1 to 11 by at least one method of the following (a) to (d):
(a) vacuum packaging;
(b) packaging together with inert gas;
(c) packaging together with an oxygen scavenger; and
(d) packaging with a wrapping material having deoxygenation function.

14. The package according to claim 13, in which a desiccant is enclosed.

## Patentansprüche

1. Biochip, aufweisend ein Substrat mit einer hydrophilen Reaktionsfläche und einen Spot aus einem zu immobilisierenden Material, das ein biologisches Material umfasst, der in der Reaktionsfläche angeordnet ist,
wobei der Spot weiter ein Verdickungsmittel und eine grenzflächenaktive Substanz umfasst,
wobei der Kontaktwinkel der hydrophilen Reaktionsfläche, gemessen durch ein statisches Kontaktwinkelmessverfahren, 1° oder mehr und weniger als 80° beträgt,
wobei das Verdickungsmittel Gellangummi, Xanthangummi, Curdlan, Pullulan, ein Guarkernmehl-Derivat, Johannisbrotkernmehl, Carrageen, Pektin, Tamarindengummi, Flohsamengummi, Dextran, Glycerin, Carboxymethylcellulose, Hydroxyethylcellulose, Hydroxymethylpropylcellulose, Lanolin, Methylcellulose, Vaseline, Polyethylenglykol, Polyvinylalkohol, Polyvinylpyrrolidon, ein Carboxylvinylpolymer, Polyvinylpyrrolidon, Polyvinylalkohol, Dextrinfettsäureester, Inulinfettsäureester oder ein Gemisch aus zwei oder mehr davon ausgewählten Arten ist,
wobei die grenzflächenaktive Substanz eine nichtionische grenzflächenaktive Substanz ist, und
wobei der Spot gebildet wird durch
einen Schritt, in dem eine Flüssigkeit, die das zu immobilisierende Material, ein Photovernetzungsmittel mit mindestens zwei photoreaktiven Gruppen in einem einzigen Molekül sowie das Verdickungsmittel und die grenzflächenaktive Substanz umfasst, immobilisieren gelassen wird, um in Form gitterartiger Spots an das Substrat zu haften; und
einen Schritt, in dem das zu immobilisierende Material, das an das Substrat haftet, darauf immobilisiert wird.

2. Biochip nach Anspruch 1, wobei die Reaktionsfläche von einer Begrenzung, die imstande ist, eine Flüssigkeit darin zurückzuhalten, umgeben ist.

3. Biochip nach Anspruch 1 oder 2, wobei das Substrat eine Oberfläche aus Harz, die die hydrophile Reaktionsfläche umfasst, aufweist.

4. Biochip nach Anspruch 3, wobei die Reaktionsfläche mit funktionellen Gruppen, die eine Polarität aufweisen, bedeckt ist, ausgewählt aus einer Hydroxylgruppe, einer Carbonylgruppe, einer Carboxylgruppe, einer Methoxygruppe, einer Peroxidgruppe und einer polaren Ethergruppe, die als Ergebnis dessen erzeugt werden, dass Bindungen, die mit Kohlenstoff des Harzes in Verbindung stehen, gespalten werden und die gespaltenen Stellen an Sauerstoff binden.

5. Biochip nach Anspruch 1 oder 2, wobei das zu immobilisierende Material auf dem Substrat mittels einer Deckschicht, die das zu immobilisierende Material zurückhält, immobilisiert ist.

6. Biochip nach Anspruch 5, wobei die Deckschicht aus einem wasserlöslichen Polymer besteht.

7. Biochip nach Anspruch 6, wobei das wasserlösliche Polymer Polyethylenglykolmethacrylat ist.

8. Biochip nach einem der Ansprüche 1 bis 7, wobei das zu immobilisierende Material ein Peptid, eine Nukleinsäure, eine Zuckerkette oder ein Gemisch aus einer oder mehr davon ausgewählten Arten ist.

9. Biochip nach einem der Ansprüche 1 bis 8, wobei das zu immobilisierende Material auf einem Mikroteilchenträger als Immobilisierungsträger immobilisiert ist, und der Immobilisierungsträger, auf dem das zu immobilisierende Material immobilisiert ist, auf der Reaktionsfläche immobilisiert ist.

10. Biochip nach einem der Ansprüche 1 bis 9, wobei die grenzflächenaktive Substanz Triton X-100, Tween 20, Tween 80 oder ein Gemisch aus zwei oder mehr davon ausgewählten Arten ist.

11. Biochip nach einem der Ansprüche 1 bis 10, wobei das Substrat ein Harzmaterial aus Polystyrol, Polypropylen, Polycarbonat und Acrylharzen ist.

12. Verfahren zur Herstellung des Biochips nach Anspruch 1, umfassend:
einen Schritt des Bildens einer hydrophilen Reaktionsfläche; und
einen Schritt des Bildens eines Spots des zu immobilisierenden Materials, das ein biologisches Material umfasst, auf der Reaktionsfläche,
wobei der Spot weiter ein Verdickungsmittel und eine grenzflächenaktive Substanz umfasst,
wobei der Kontaktwinkel der hydrophilen Reaktionsfläche, gemessen durch ein statisches Kontaktwinkelmessverfahren, 1° oder mehr und weniger als 80° beträgt,
wobei das Verdickungsmittel Gellangummi, Xanthangummi, Curdlan, Pullulan, ein Guarkernmehl-Derivat, Johannisbrotkernmehl, Carrageen, Pektin, Tamarindengummi, Flohsamengummi, Dextran, Glycerin, Carboxymethylcellulose, Hydroxyethylcellulose, Hydroxymethylpropylcellulose, Lanolin, Methylcellulose, Vaseline, Polyethylenglykol, Polyvinylalkohol, Polyvinylpyrrolidon, ein Carboxylvinylpolymer, Polyvinylpyrrolidon, Polyvinylalkohol, Dextrinfettsäureester, Inulinfettsäureester oder ein Gemisch aus zwei oder mehr davon ausgewählten Arten ist,
wobei die grenzflächenaktive Substanz eine nichtionische grenzflächenaktive Substanz ist, und
wobei der Schritt des Bildens eines Spots umfasst:
einen Schritt, in dem eine Flüssigkeit, die das zu immobilisierende Material, ein Photovernetzungsmittel mit mindestens zwei photoreaktiven Gruppen in einem einzigen Molekül sowie das Verdickungsmittel und die grenzflächenaktive Substanz umfasst, immobilisieren gelassen wird, um in Form gitterartiger Spots an das Substrat zu haften; und
einen Schritt, in dem das zu immobilisierende Material, das an das Substrat haftet, darauf immobilisiert wird.

13. Verpackung, dargestellt durch Verpacken des Biochips nach einem der Ansprüche 1 bis 11 durch mindestens ein Verfahren aus den nachstehenden (a) bis (d):
(a) Vakuumverpacken;
(b) Verpacken zusammen mit einem Inertgas;
(c) Verpacken zusammen mit einem Sauerstofffänger; und
(d) Verpacken mit einem Umhüllungsmaterial, das eine sauerstoffentziehende Wirkung aufweist.

14. Verpackung nach Anspruch 13, in der ein Trockenmittel eingeschlossen ist.

## Revendications

1. Biopuce présentant un substrat doté d'une zone de réaction hydrophile et un point de matière à immobiliser comprenant une matière biologique qui est disposée dans la zone de réaction,
dans laquelle le point comprend en outre un épaississant et un tensioactif,
dans laquelle l'angle de contact de la zone réactionnelle hydrophile mesuré par une méthode de mesure d'angle de contact statique fait 1° ou plus et moins de 80°, dans laquelle l'épaississant est la gomme gellane, la gomme de xanthane, le curdlan, le pullulan, un dérivé de la gomme de guar, la gomme de fève de caroube, la carraghénine, la pectine, la gomme de tamarin, la gomme de psyllium, le dextrane, la glycérine, la carboxyméthylcellulose, l'hydroxyéthylcellulose, l'hydroxyméthylpropyl-cellulose, la lanoline, la méthylcellulose, la Vaseline, le polyéthylèneglycol, l'alcool de polyvinyle, la polyvinylpyrrolidone, un polymère carboxylvinyle, la polyvinylpyrrolidone, l'alcool de polyvinyle, l'ester gras d'acide de dextrine, l'ester gras d'acide d'inuline ou un mélange de deux types ou plus de ceux-ci,
dans laquelle le tensioactif est un tensioactif non ionique, et
dans laquelle le point est formé par
une étape où on l'on permet à un liquide comprenant la matière à immobiliser, un agent de photoréticulation présentant au moins deux groupements photoréactifs dans une seule molécule et l'épaississant et le surfactant, à adhérer, sous la forme de points ressemblant à un treillis, au substrat, et
une étape d'immobilisation de la matière à immobiliser adhérant au substrat dessus.

2. Biopuce selon la revendication 1, dans laquelle la zone réactionnelle est entourée par une limite à même de retenir un liquide y contenu.

3. Biopuce selon la revendication 1 ou 2, dans laquelle le substrat a une surface fabriquée en résine comprenant la zone réactionnelle hydrophile.

4. Biopuce selon la revendication 3, dans laquelle la zone réactionnelle est recouverte par des groupements fonctionnels présentant une polarité sélectionnés parmi un groupement hydroxyle, un groupement carbonyle, un groupement carboxyle, un groupement méthoxy, un groupement peroxyde et un groupement éther polaire, lesquels sont générés comme une conséquence que les liaisons associées avec le carbone de la résine sont clivées et les sites clivés se lient à l'oxygène.

5. Biopuce selon la revendication 1 ou 2, dans laquelle la matière à immobiliser est immobilisée sur le substrat via une couche de revêtement retenant la matière à immobiliser.

6. Biopuce selon la revendication 5, dans laquelle la couche de revêtement consiste en un polymère soluble dans l'eau.

7. Biopuce selon la revendication 6, dans laquelle le polymère soluble dans l'eau est le méthacrylate de polyéthylèneglycol.

8. Biopuce selon l'une quelconque des revendications 1 à 7, dans laquelle la matière à immobiliser est un peptide, un acide nucléique, une chaîne de sucre ou un mélange d'un ou de plusieurs types sélectionnés parmi ceux-ci.

9. Biopuce selon l'une quelconque des revendications 1 à 8, dans laquelle la matière à immobiliser est immobilisée sur un support de microparticule en tant que support d'immobilisation, et le support d'immobilisation sur lequel la matière à immobiliser est immobilisée, est immobilisée sur la zone réactionnelle.

10. Biopuce selon l'une quelconque des revendications 1 à 9, dans laquelle le tensioactif est le Triton X-100, le Tween 20, le Tween 80 ou un mélange de deux types, ou plus, sélectionnés parmi ceux-ci.

11. Biopuce selon l'une quelconque des revendications 1 à 10, dans laquelle le substrat est une quelconque matière de résine de polystyrène, polypropylène, polycarbonate et des résines acryliques.

12. Procédé de production de la biopuce selon la revendication 1, comprenant :
une étape où l'on forme une zone réactionnelle hydrophile; et
une étape où l'on forme un point de la matière à immobiliser comprenant une matière biologique sur la zone réactionnelle,
dans laquelle le point comprend un épaississant et un tensioactif,
dans laquelle l'angle de contact de la zone réactionnelle hydrophile mesuré par une méthode de mesure d'angle de contact statique fait 1° ou plus et moins de 80°,
dans laquelle l'épaississant est la gomme gellane, la gomme de xanthane, le curdlan, le pullulan, un dérivé de la gomme de guar, la gomme de fève de caroube, la carraghénine, la pectine, la gomme de tamarin, la gomme de psyllium, le dextrane, la glycérine, la carboxyméthylcellulose, l'hydroxyéthylcellulose, l'hydroxyméthylpropyl-cellulose, la lanoline, la méthylcellulose, la Vaseline, le polyéthylèneglycol, l'alcool de polyvinyle, la polyvinylpyrrolidone, un polymère carboxylvinyle, la polyvinylpyrrolidone, l'alcool de polyvinyle, l'ester gras d'acide de dextrine, l'ester gras d'acide d'inuline ou un mélange de deux types ou plus de ceux-ci,
dans laquelle le tensioactif est un surfatif non ionique, et
dans laquelle l'étape de formation d'un point comprend :
une étape où l'on permet à un liquide comprenant la matière à immobiliser, un agent de photoréticulation présentant au moins deux groupements photoréactifs dans une seule molécule et l'épaississant et le tensioactif, à adhérer, sous la forme de points ressemblant à un treillis, au substrat, et
une étape où l'on immobilise de la matière à immobiliser adhérant au substrat dessus.

13. Conditionnement préparé par conditionnement de la biopuce selon l'une quelconque des revendications 1 à 11 par au moins un procédé des (a) à (d) suivants :
(a) conditionnement sous vide ;
(b) conditionnement avec gaz inerte ;
(c) conditionnement avec éliminateur d'oxygène ; et
(d) conditionnement avec matière d'emballage présentant une fonction de désoxygénation.

14. Conditionnement selon la revendication 13, lequel contient un agent dessicatif.
